# EUROPEAN PATENT APPLICATION

(11) **EP 4 516 205 A1**
(43) Date of publication of application: **05.03.2025**
(21) Application number: 24197251.2
(22) Date of filing: 29.08.2024
(51) Int. Cl.: A61B 3/08, A61B 3/103

(54) **OPHTHALMIC EXAMINATION APPARATUS AND OPHTHALMIC EXAMINATION METHOD**

(30) Priority: 31.08.2023 JP 2023141156
(71) Applicant: OSAKA UNIVERSITY, Suita-shi Osaka 565-0871 (JP); TOPCON CORPORATION, Tokyo 174-8580 (JP)
(72) Inventor: FUJIKADO, Takashi, Osaka, 565-0871 (JP); TATARA, Yoko, Tokyo, 174-8580 (JP); YUKIMORI, Takafumi, Tokyo, 174-8580 (JP); SAIKA, Makoto, Tokyo, 174-8580 (JP); NORO, Ryoka, Tokyo, 174-8580 (JP)
(74) Representative: Louis Pöhlau Lohrentz

(57) **Abstract**

An ophthalmic examination apparatus (100) includes a visual target projection system (4) that includes a visual target presentation portion that presents a fixed visual target to left and right subject eyes (EL, ER), an objective measurement optical system (6, 7) that objectively measures eye characteristics of the left and right subject eyes (EL, ER), and a controller (140) that controls each portion of the apparatus, wherein the controller (140) measures the eye characteristics of the left and right subject eyes (EL, ER) with the objective measurement optical system (6, 7) and acquires an objective refraction value as objective measurement information when fusion is attempted with left and right subject eyes (EL, ER) with respect to a change in the convergence distance.

## Description

### CROSS-REFERENCE TO RELATED APPLICATION

The present application is based on and claims priority from Japanese Patent Application No. 2023-141156 filed on August 31, 2023, the disclosure of which is hereby incorporated by reference in its entirety.

### FILED OF THE INVENTION

The present disclosure relates to an ophthalmic examination apparatus and an ophthalmic examination method.

### BACKGROUND

Techniques regarding ophthalmic examinations have been disclosed in some documents such as JP 2022-038942 A, JP 2021-019957 A, JP 2020-069201 A, JP 2019-069049 A, and JP 2019-063238 A. JP 2022-038942 A has disclosed a technique to provide optometry information that allows users to prescribe a correction lens for a subject (patient) with heterophoria. JP 2021-019957 A has described a technique to provide an ophthalmic measurement apparatus that allows users to examine eyes in a state closer to natural vision where the binocular fusion of the eye is appropriately maintained. JP 2020-069201 A has disclosed a technique to provide an optometry system that allows users to check the position of the line of sight of the subject eye and examine eyes more appropriately. JP 2019-069049 A has disclosed a technique to provide an ophthalmic apparatus that allows users to conduct an objective examination on a state related to an eye position. JP 2019-063238 A has disclosed a technique to provide an ophthalmic apparatus that allows users to conduct objective examination on a state related to an eye position. JP 2018-047050 A has disclosed a technique that allows users to check a fusion state more easily of a subject who opens both eyes and measure the optical characteristics of the subject's eyes in higher accuracy.

### SUMMARY

When viewing a usual, three-dimensional (3D) video, eyes experience a discrepancy between convergence, which is the eye movement involved in fusing two images into as a single perception with both eyes, and accommodation, which is an adjustment function involved in focusing the images with both eyes. That is, the convergence and the accommodation involve therebetween a range of the convergence (relative convergence) where the fusion of two visual targets into one with both eyes can be achieved while the accommodation is maintained constant accommodation, and a range of the accommodation (relative adjustment) where the fusion can be achieved while the convergence is maintained constant. For this reason, exceeding the ranges of the relative convergence or the relative adjustment will trigger convergence movement (accommodative convergence) cooperating with the accommodative response or accommodative operation (convergence accommodation) cooperating with the convergence. Here, the term "convergence" refers to an eye movement of a subject (patient) in a direction in which both eyes approach each other, between eye movements in which both eyes move in different directions when the subject changes the line of sight from an object to another object at a different distance (depth direction to subject).

Meanwhile, with reference to JP 2022-038942 A, JP 2021-019957 A, JP 2020-069201 A, JP 2019-069049 A, or JP 2019-063238 A, these prior-art documents have disclosed no configuration for producing the discrepant state between the convergence and the accommodation by applying the convergence stimulus when the system arranges two visual targets at positions at a certain distance from the subject's eyes that see the two visual targets fused with both eyes. For this reason, the prior-art disclosures fail to assess the subject's eyes whether they can easily adapt themselves to stereoscopic vision, in which a discrepancy between convergence and accommodation occurs.

The present disclosure has been made in view of the above problems, and an object of the present disclosure aims to provide an ophthalmic examination apparatus and an ophthalmic examination method capable of evaluating whether the left and right subject eyes of the subject can easily adapt themselves to stereoscopic vision in which a discrepancy between convergence and accommodation occurs.

An ophthalmic examination apparatus of the present disclosure includes: a visual target projection system including a visual target presentation portion that presents a fixed visual target to left and right subject eyes to be examined; an objective measurement optical system that objectively measures eye characteristics of the left and right subject eyes; and a controller that controls each portion of the apparatus. The fixed visual target is a visual target for a left eye and a visual target for a right eye that are respectively projected onto the left and right subject eyes and have the same pattern. The visual mark presentation portion presents the visual target for the left eye and the visual target for the right eye at visual target positions separated from the left and right subject eyes by a certain examination distance. The visual target projection system prescribes a state in which a convergence distance from positions of the left and right subject eyes to a convergence position where two lines of sight intersect each other is changed by changing an angle between the lines of sight from the left and right subject eyes toward the visual target for the left eye and the visual target for the right eye. When fusion is attempted with the left and right subject eyes to a change in the convergence distance, the controller measures the eye characteristics of the left and right subject eyes with the objective measurement optical system to acquire an objective refraction value as objective measurement information.

An ophthalmic examination method of the present disclosure includes a complete correction prescription step, a visual target presentation step, a convergence distance control step, an adjustment reaction amount measurement step, and a determination step. The complete correction prescription step assumes the left and right subject eyes as a state in which a complete correction value in subjective examination is prescribed. The visual target presentation step presents a visual target for the left eye and a visual target for the right eye having a same pattern in the visual mark presentation portion at visual target positions separated from the left and right subject eyes by a certain examination distance following the prescription of the complete correction value. The convergence distance control step controls an angle between lines of sight from the left and right subject eyes toward the visual target for the left eye and the visual target for the right eye to prescribe a state in which a convergence distance from positions of the left and right subject eyes to a convergence position where the two lines of sight intersect each other is changed. The accommodation reaction amount measurement step is executed in parallel with the control of the convergence distance and measures the eye characteristics of the left and right subject eyes with the objective measurement optical system when fusion is attempted with the left and right subject eyes to the change of the convergence distance to acquire an objective refraction value as objective measurement information. The determination step determines adaptation abilities of the left and right subject eyes to stereoscopic vision based on the objective refraction value.

### BRIEF DESCRIPTION OF DRAWINGS

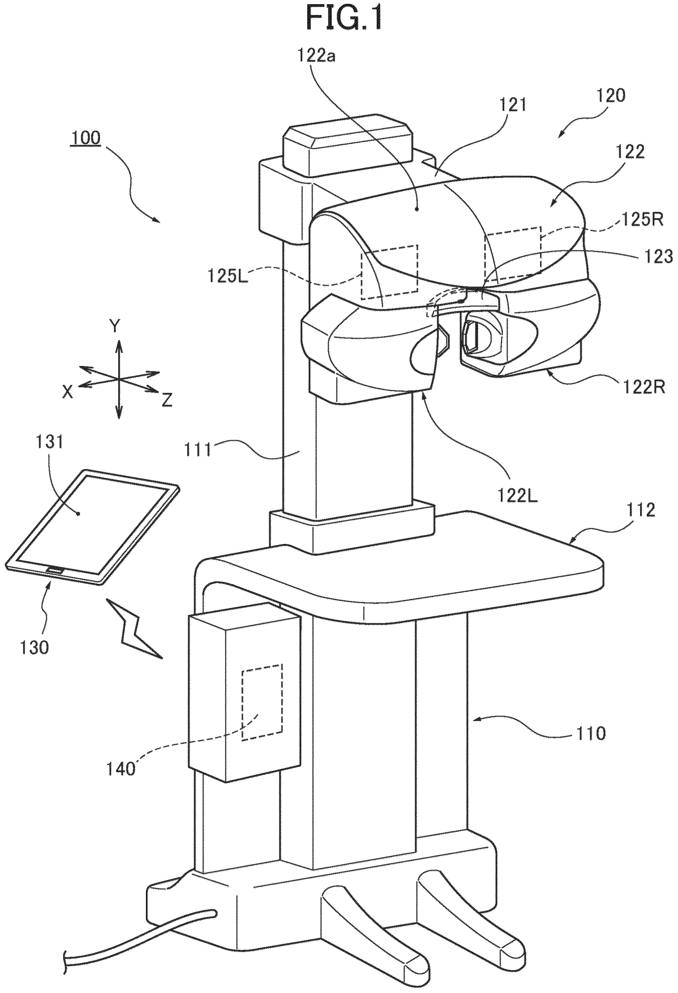

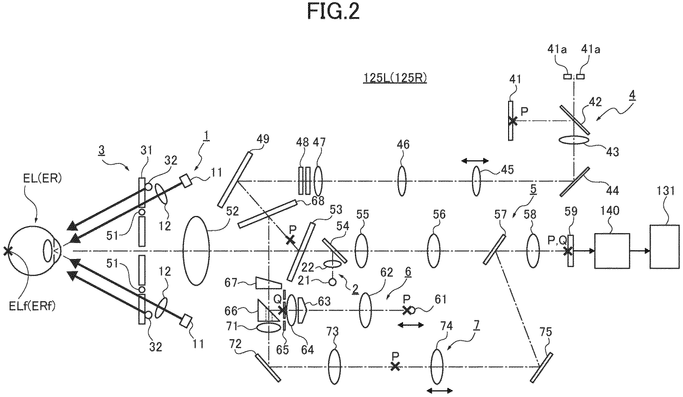

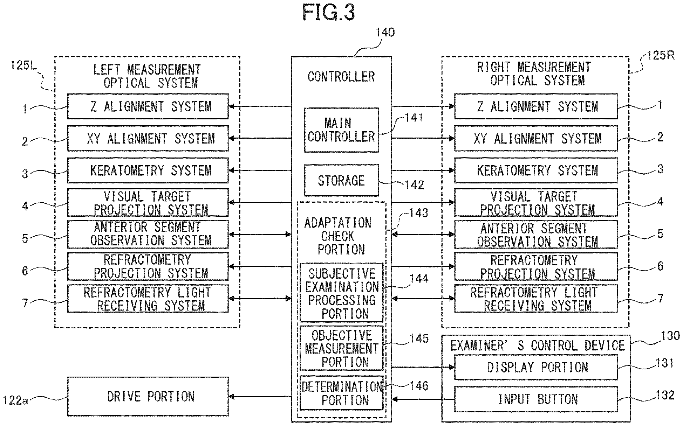

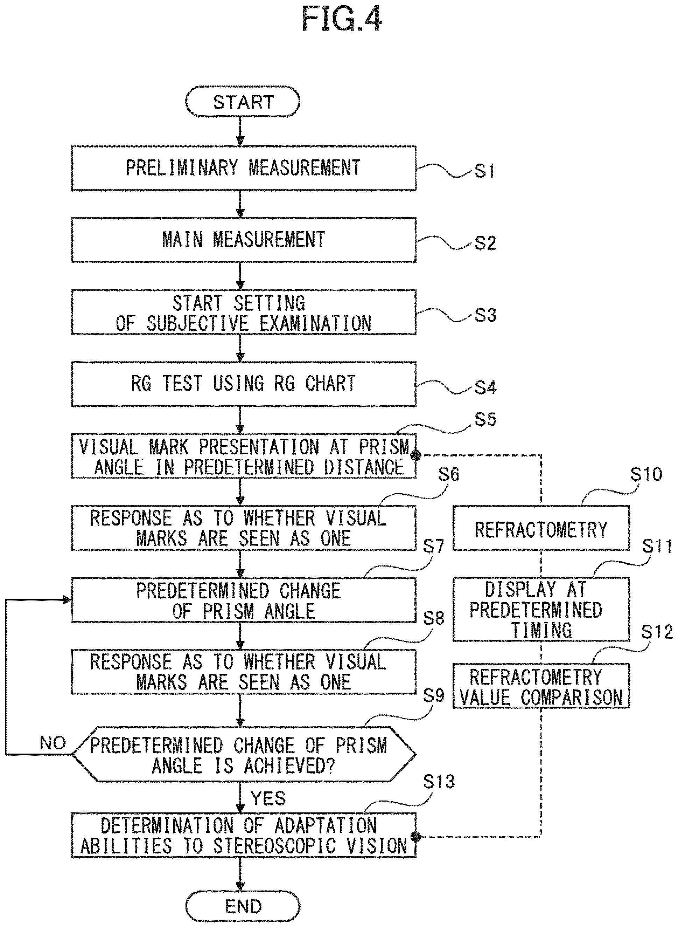

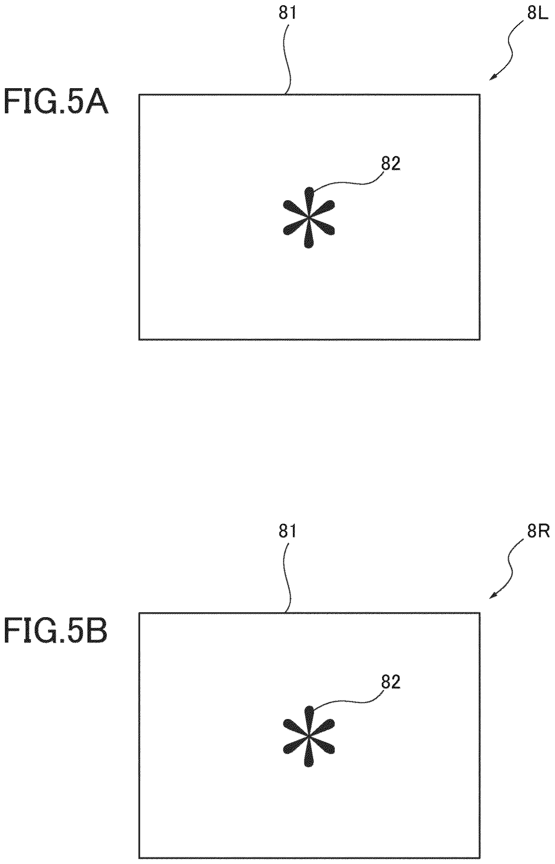

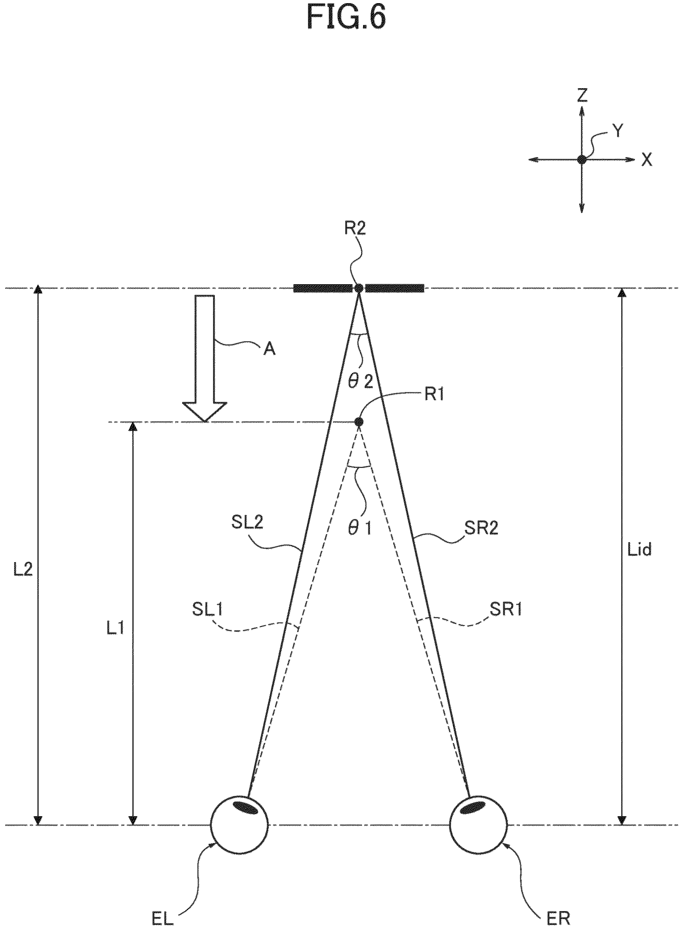

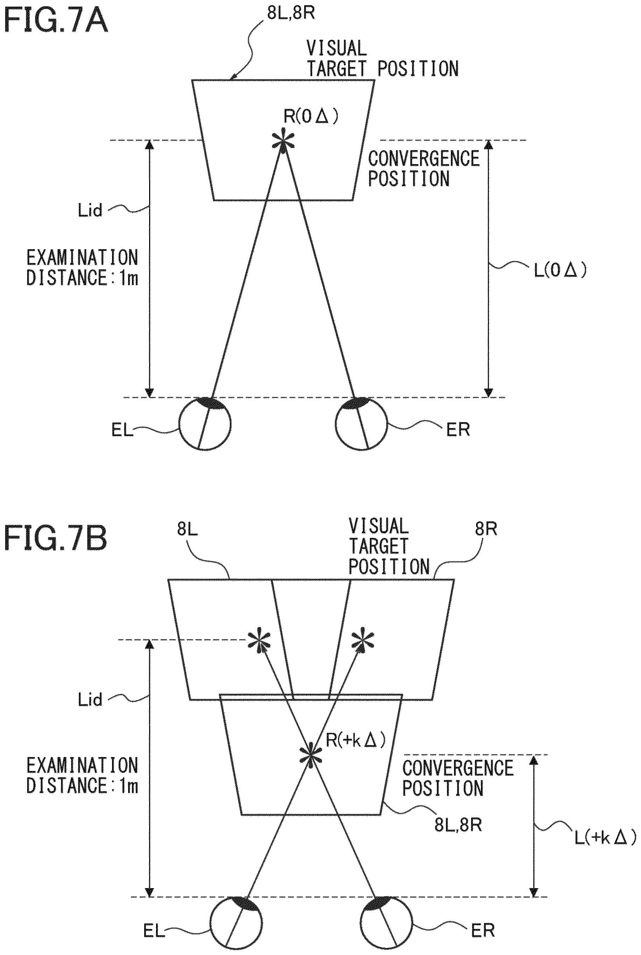

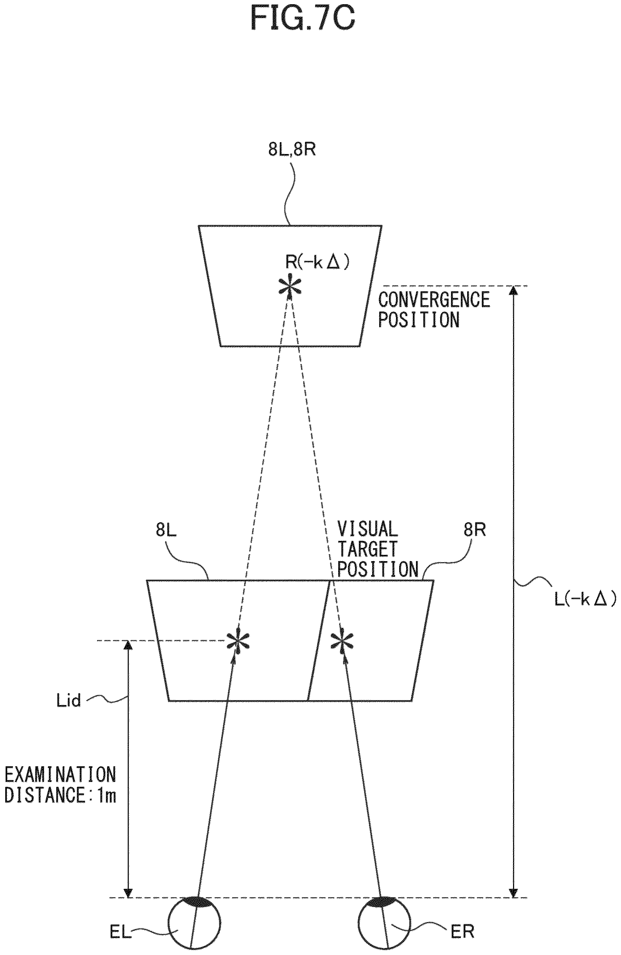

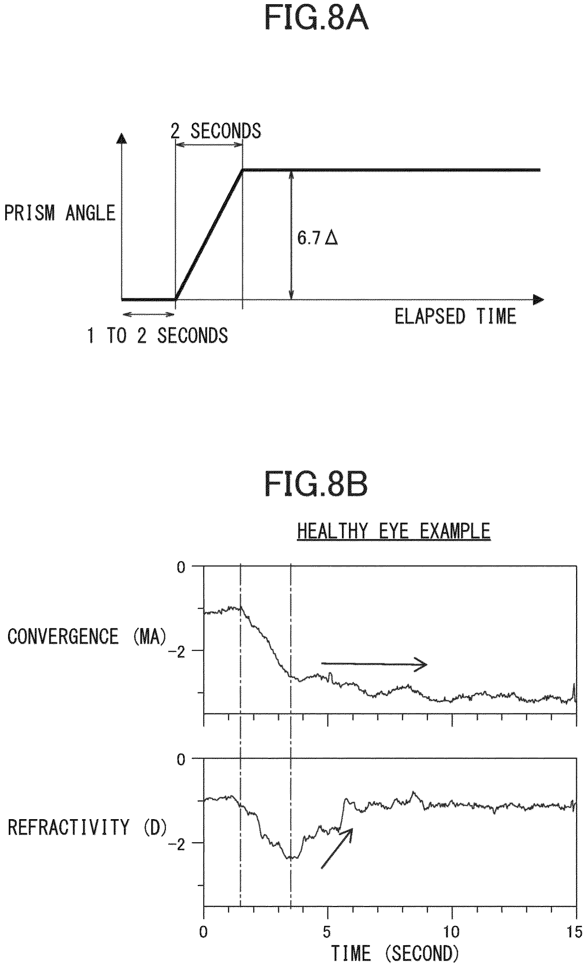

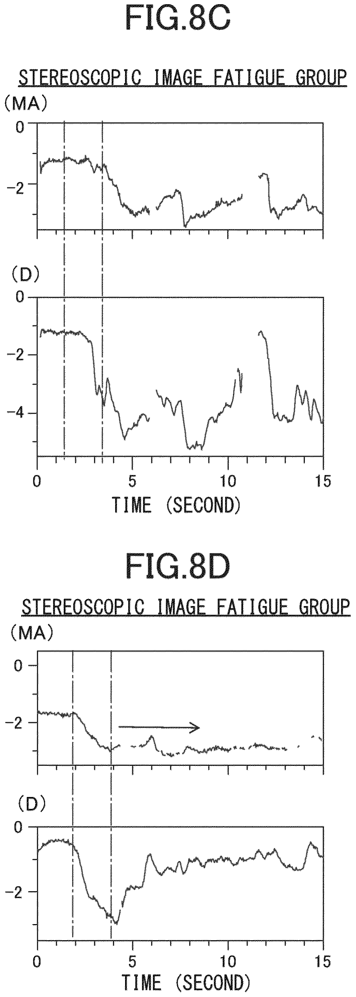

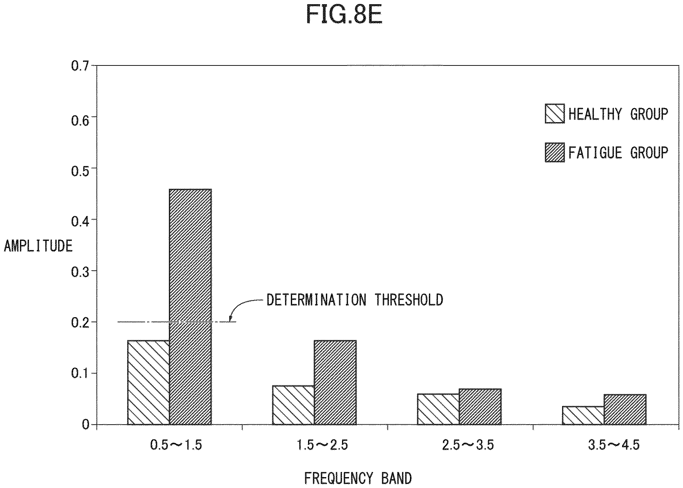

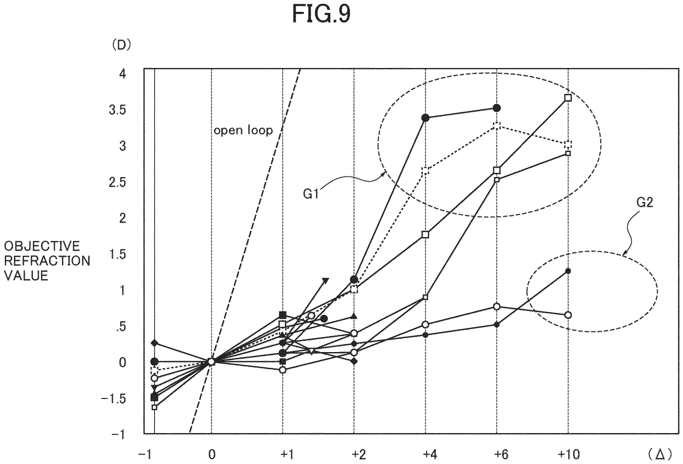

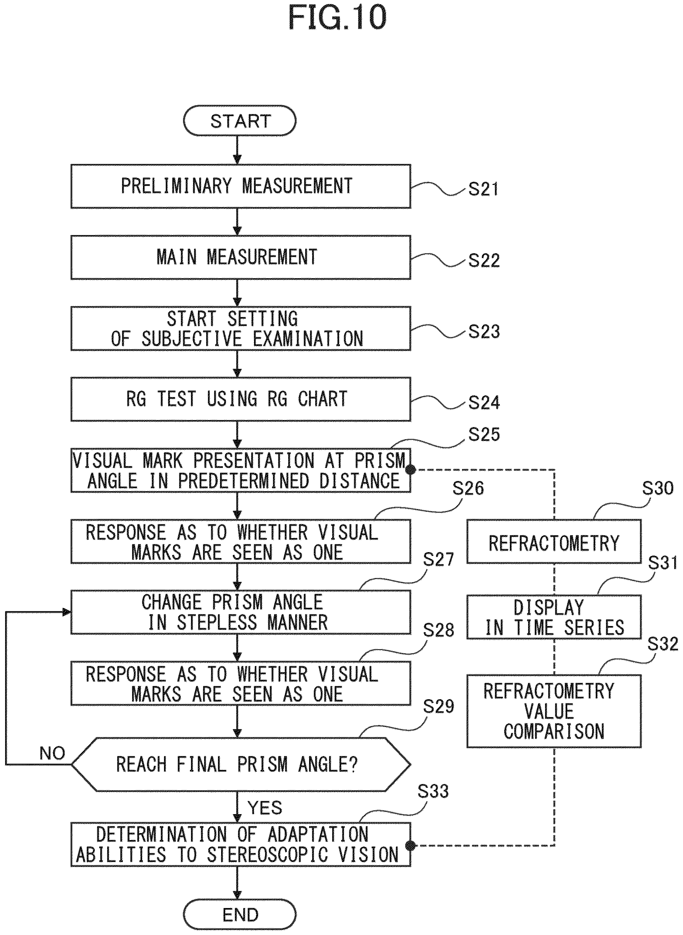

FIG. 1 is an external perspective view of an ophthalmic examination apparatus of Example 1. FIG. 2 illustrates a schematic configuration example of a left measurement optical system in the ophthalmic examination apparatus of Example 1. FIG. 3 illustrates a control block configuration of the ophthalmic examination apparatus according to Example 1. FIG. 4 is a flowchart showing a process sequence of an ophthalmic examination method of Example 1. FIG. 5A illustrates an example of a left eye projection visual target. FIG. 5B illustrates an example of a right eye projection visual target. FIG. 6 is an explanatory view illustrating a convergence distance to a convergence position where two lines of sight intersect each other. FIG. 7A illustrates an example in which the convergence distance to the convergence position matches with an examination distance. FIG. 7B illustrates an example in which the convergence distance to the convergence position is shorter than the examination distance. FIG. 7C illustrates an example in which the convergence distance to the convergence position is farther than the examination distance. FIG. 8A is a prism angle time chart in measurement of convergence and accommodation. FIG. 8B illustrates a measurement result example of convergence and a refractivity (accommodation) by a healthy eye example. FIG. 8C illustrates measurement result example 1 of convergence and a refractivity (accommodation) by a fatigue group. FIG. 8D illustrates measurement result example 2 of convergence and a refractivity (accommodation) by a fatigue group. FIG. 8E illustrates an analysis result example of a refraction time change frequency by a healthy group and a fatigue group. FIG. 9 illustrates a relationship characteristic between a prism angle and an objective refraction value for which plurality of subject eyes are measured. FIG. 10 is a flowchart showing a process sequence of an ophthalmic examination method of Example 2.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

The embodiments for implementing an ophthalmic examination apparatus and an ophthalmic examination method of the present disclosure are described below with reference to Examples 1 and 2 shown in the drawings.

The ophthalmic examination apparatus of the Examples 1 and 2 is a both-eye open type apparatus that can simultaneously measure the eye characteristics of both eyes at the same time with an examinee opening both eyes. This ophthalmic examination apparatus can also measure the eye characteristics of each eye at a time by shielding one of the eyes or turning off a fixed visual target for the one of the eyes. In addition, the ophthalmic examination apparatus is an objective measurement apparatus with subjective examination functions and the objective measurement apparatus includes a visual target presentation function, a phoropter function, and an automatic refraction and keratometry function. This ophthalmologic apparatus allows an examiner to objectively and subjectively measure the eye characteristics of the subject eyes with arbitrary objective examination and arbitrary subjective examination.

The objective examination that the ophthalmic examination apparatus can execute includes measurement for acquiring the eye characteristics and photographing for acquiring the image of the subject eye. The objective examination includes the refractive power measurement (refractometry measurement), the corneal shape measurement (keratometry measurement), the intraocular pressure measurement, the ocular fundus photographing, the tomogram imaging using optical coherence tomography (OCT) (OCT imaging), and the measurement using OCT. In addition, the subjective examination presents, for example, a visual target to the subject, and measures information (eye characteristics) on the subject eye based on the response of the subject to the presented visual target, for example. The subjective examination includes subjective refraction measurement such as the far-point examination, the intermediate-point examination, the near-point examination, the contrast examination, and the glare examination, and the visual field examination.

### Example 1

[Overall Configuration of Apparatus (FIG. 1)] FIG. 1 describes an overall configuration of an ophthalmic examination apparatus 100. As illustrated in FIG. 1, the ophthalmic examination apparatus 100 includes a support base 110, a measurement unit 120, an examiner's control device 130, and a controller 140. Axes used in the drawings and the description, when viewed from the subject, includes an axis in the left-right direction (horizontal axis) as the X axis, an axis in the up-down direction (vertical axis) as the Y axis, and an axis perpendicular to the X axis and the Y axis in the depth direction (front-back axis) as the Z axis.

The support base 110 includes a support column 111 standing upright from the floor surface and an optometry table 112 supported by the support column 111. The optometry table 112 is a support on which devices and tools used for optometry such as the examiner's control device 130 are placed or which supports the posture of the subject. The optometry table 112 may have a fixed position in the Y-axis direction (height position) or may be supported on the support column 111, which can adjust the position in the Y-axis direction (height position).

The measurement unit 120 includes an arm 121, a measurement head 122, and a forehead rest 123. The arm 121 includes one end supported by the distal end portion of the support column 111, the other end extending from the support column 111 to the front side (subject side) in the Z-axis direction, and the distal end portion to which the measurement head 122 is attached. As a result, the measurement head 122 suspends suspended from the support column 111 via the arm 121 above the optometry table 112. Also, the arm 121 is configured to move in the Y-axis direction with respect to the support column 111. Note that the arm 121 is also configured to move in the X-axis direction or the Z-axis direction with respect to the support column 111.

The measurement head 122 measures the eye characteristics of the subject eye. The measurement head 122 includes a drive portion 122a, a pair of a left measurement head portions 122L and a right measurement head portion 122R provided below the drive portion 122a, and the forehead rest 123. Here, the left measurement head portion 122L and the right measurement head portion 122R individually correspond to the left and right subject eyes of the subject.

The drive portion 122a is a mechanism that individually drives each of the left measurement head portion 122L and the right measurement head portion 122R to move in the horizontal direction (X-axis direction), move in the vertical direction (Y-axis direction), rotate about the X-axis, or rotate about the Y-axis.

The left measurement head portion 122L includes a left measurement optical system 125L. The left measurement optical system 125L presents a visual target to the subject eye on the left side of the subject (hereinafter, "left subject eye") and measures the eye characteristics of the left subject eye in a state where an arbitrary spherical power (correction degree) is set. The right measurement head portion 122R includes a right measurement optical system 125R. The right measurement optical system 125R presents a visual target to a subject eye on the right side of the subject (hereinafter, "right subject eye") and measures the eye characteristics of the right subject eye in a state where an arbitrary spherical power (correction degree) is set. The measurement results by the left measurement optical system 125L and the right measurement optical system 125R are hereinafter referred to as objective measurement information. Note that detailed configurations of the left measurement optical system 125L and the right measurement optical system 125R are described below in "Configuration of Optical System".

The forehead rest123 is provided in the measurement unit 120 and is disposed between the left measurement head portion 122L and the right measurement head portion 122R. The forehead rest 123 supports the face of the subject by bringing a part (forehead) of the face of the subject into contact during the measurement of the eye characteristics. That is, the subject facing the optometry table 112 presses the subject's own forehead against the forehead rest 123 to stabilize the orientation and position of the face so as not to move. Moving the arm 121 in the Y-axis direction with respect to the support column 111 adjusts the position of the forehead rest 123 in the height direction.

The examiner's control device 130 is an information processing device that receives input operations by the examiner and outputs control signals to the controller 140. The examiner's control device 130 is, for example, a tablet terminal and a smartphone and is separated from the measurement unit 120 and is able to be carried by the examiner. Note that the examiner's control device 130 may be a notebook personal computer or a desktop personal computer or may be a dedicated controller provided in the ophthalmic examination apparatus 100. The examiner's control device 130 can exchange information with the controller 140 via wireless communication or network communication.

As illustrated in FIG. 1, the examiner's control device 130 includes a display portion 131 and an operation-side controller (not illustrated). The display portion 131 includes a touch panel display provided on the surface of the examiner's control device 130, and, for example, screen display sets an input button 132 (see FIG. 3). The operation-side controller includes a microcomputer equipped in the examiner's control device 130. The operation-side controller controls images to be displayed on the display portion 131 based on measurement results and/or detection results transmitted from the controller 140. Further, the operation-side controller outputs control signals corresponding to an operation on the displayed input button 132, for example, to the controller 140.

[Configuration of Optical System (FIG. 2)] Detailed configurations of the left measurement optical system 125L and the right measurement optical system 125R are described below with reference to FIG. 2. Note that the left measurement optical system 125L and the right measurement optical system 125R have the same configuration. Accordingly, in the present specification, the illustration and the description of the right measurement optical system 125R corresponding to a right subject eye ER are omitted, and only the left measurement optical system 125L corresponding to a left subject eye EL is described.

The left measurement optical system 125L is an optical system that presents a visual target to the left subject eye EL and measures the left subject eye. As illustrated in FIG. 2, the left measurement optical system 125L includes a Z-alignment system 1, an XY-alignment system 2, a keratometry system 3, a visual target projection system 4, an anterior segment observation system 5, a refractometry projection system 6, and a refractometry light receiving system 7. Note that a "fundus conjugate position P" used in the following description means position optically and substantially conjugate with a fundus ELf of the left subject eye EL when alignment is completed and means a position optically conjugate with the fundus ELf of the left subject eye EL or the vicinity thereof. A "pupil conjugate position Q" is a position optically and substantially conjugate with the pupil of the left subject eye EL when alignment is completed and means a position optically conjugate with the pupil of the left subject eye EL or the vicinity thereof.

The Z-alignment system 1 projects light (infrared light) for aligning in the optical axis direction (front-back direction = Z-axis direction) of the anterior segment observation system 5 to the left subject eye EL. The light projected from a Z-alignment light source 11 is converted into a parallel luminous flux by using a projection lens 12 and projected onto the cornea of the left subject eyeEL through an alignment hole formed in a keratometric plate 31. Based on the bright spot projected on the cornea, the controller 140 or the examiner controls the drive portion 122a so that the ratio of a distance between the two-point images with the Z-alignment light source 11 on an imaging element 59 and the diameter of a keratometric ring image falls within a predetermined range, and moves the left measurement optical system 125L in the Z-axis direction.

The XY-alignment system 2 irradiates the left subject eye EL with light (infrared light) for performing alignment in the X-axis direction and the Y-axis direction orthogonal to the optical axis (Z-axis) of the anterior segment observation system 5. The XY-alignment system 2 includes an XY-alignment light source 21 and a projection lens 22, which are disposed in an optical path that is branched from that of the anterior segment observation system 5 with a half mirror 54. The light output from the XY-alignment light source 21 transmits through the projection lens 22, reflects with the half mirror 54, and projects onto the left subject eye EL through the anterior segment observation system 5. Reflected light from the cornea of the left subject eye EL is guided to the imaging element 59 through the anterior segment observation system 5. Note that the alignment method in each of XYZ-directions is not limited to the method using the Z-alignment system 1 or the XY-alignment system 2, and any method may be used as long as the position of the subject eye E can be measured, such as a method using a stereo camera for the alignment provided with the ophthalmic examination apparatus 100.

Here, the reflected light from the cornea of the left subject eye EL forms an image on the imaging element 59 as a bright spot image, which is included in a part of an anterior segment image. The controller 140 controls the display portion 131 to display the anterior segment image, which includes the bright spot image, and an alignment mark. The anterior segment image (bright spot image) and alignment mark are used as follows: for the manual XY-alignment, the examiner uses the examiner's control device 130 to control the drive portion 122a to move the left measurement optical system 125L in the X-axis direction and the Y-axis direction so as to put the bright spot image in the alignment mark; for the automatic XY-alignment, the controller 140 controls the drive portion 122a to move the left measurement optical system 125L in the X-axis direction and the Y-axis direction so as to eliminate the displacement of the bright spot image relative to the alignment mark.

The keratometry system 3 projects a ring-shaped luminous flux (infrared light) onto the cornea to measure the shape of the cornea of the left subject eye EL. The keratometric plate 31 is disposed at a position between an objective lens 52 and the left subject eye EL, and a keratometric ring light source 32 is provided on the back surface side (objective lens 52 side). The keratometric ring light source 32 projects a ring-shaped luminous flux onto the cornea of the left subject eye EL by illuminating the keratometric plate 31 with light from the back surface. Reflected light (keratometric ring image) from the cornea of the left subject eye EL is detected as well as the anterior segment image with the imaging element 59. The controller 140 calculates corneal shape parameters representing the shape of the cornea with a known calculation based on the keratometric ring image.

The visual target projection system 4 presents various visual targets, such as a fixed visual target and a visual target for the subjective examination, to the left subject eye EL. The visual target projection system 4 includes a display 41, a half mirror 42, a relay lens 43, a reflection mirror 44, a focusing lens 45, a relay lens 46, a field lens 47, a variable cross cylinder lens (VCC) 48, and a reflection mirror 49. The visual target projection system 4 shares a dichroic mirror 68 with the refractometry projection system 6. The visual target projection system 4 shares a dichroic mirror 53 and the objective lens 52 with the anterior segment observation system 5. Furthermore, the visual target projection system 4 includes at least two glare light sources 41a, which illuminate the left subject eye EL with glare light, at a position around the optical axis and on an optical path different from an optical path to the display 41, which displays the visual target for the left eye.

The light projected from the display 41 reflects on the half mirror 42, transmits through the relay lens 43, reflects on the reflection mirror 44, and transmits through the focusing lens 45. The light transmitted through the focusing lens 45 transmits through the relay lens 46, transmits through the VCC 48 after the field lens 47 aligns traveling direction, reflects on the reflection mirror 49, transmits through the dichroic mirror 68, and reflects on the dichroic mirror 53. The light reflected with the dichroic mirror passes through the objective lens 52 and is projected onto the fundus ELf.

The display 41 is configured to display various visual targets including a fixed visual target or a point visual target as a visual target for fixing a line of sight for an objective examination or for cloud fogging to the left subject eye EL, and a subjective examination visual target for subjectively examining the eye characteristics (visual acuity value, dioptric power for long distance, dioptric power for short distance, and the like) of the left subject eye EL. The display 41 may be fabricated with a liquid crystal display or an organic EL display. Then, the visual targets to be projected on the subject eye E can be arbitrarily created by using a still image or a moving image, which can be used as a chart icon that can be selected on a chart page. The display 41 is configured to display a selected created visual target of the still image or a created visual target of the moving image from the chart page. The display 41 is provided at the fundus conjugate position P on the optical path of the visual target projection system 4.

The focusing lens 45 is moved forward and backward in the optical axis direction with a drive motor (not illustrated) controlled with the controller 140. Controlling to move the focusing lens 45 in a direction toward the left subject eye EL allows the controller 140 to change the spherical power of the left subject eye EL to the negative diopter side (-D side). Also, controlling to move the focusing lens 45 in a direction away from the left subject eye EL allows the controller 140 to change the spherical power of the left subject eye EL to the positive diopter side (+D side). Furthermore, controlling to move the focusing lens 45 forward and backward allows the controller 140 to change the examination distance between the left subject eye EL and the visual target presentation position. Here, with the controlling of the focusing lens 45, a refractometry light source 61 and a focusing lens 74 are controlled to move together.

For the subjective examination, the controller 140controls the examination distance or the spherical power of the left subject eye EL by moving the focusing lens 45 in the optical axis direction based on the result of the objective measurement. Then, the controller 140 displays a predetermined visual target selected by the examiner or the controller 140 on the display 41. As a result, the visual target is presented to the subject at a predetermined examination distance with respect to the left subject eye EL adjusted to a predetermined spherical power. Also, the controller 140 is configured to receive a subjective response the subject has made to the visual target. For example, for visual acuity measurement, the examiner or the controller 140 is configured to present the visual target, such as the Landolt's ring, to the subject and receive the subjective response to select the alternative mark, which are conducted repeatedly, to determine the visual acuity value of the subject.

The anterior segment observation system 5 observes the anterior segment of the left subject eye EL and captures the anterior segment. An anterior segment illumination light source 51 illuminates light (for example, infrared light) onto the anterior segment of the left subject eye EL. The light reflected on the anterior segment of the left subject eye EL passes through the objective lens 52, transmits through the dichroic mirror 53, transmits through the half mirror 54, passes through a relay lens 55 and a relay lens 56, and transmits through a dichroic mirror 57. The light transmitted through the dichroic mirror 57 forms an image on the imaging surface of the imaging element 59 by using an image forming lens 58. The imaging element 59 captures images to output a signal at a predetermined rate. The output (video signal) of the imaging element 59 is input to the controller 140. The controller 140 displays an anterior segment image (moving image), which is based on the image signal as an output of the imaging element 59, on the display portion 131 of the examiner's control device 130. The imaging surface of the imaging element 59 in the optical system of the anterior segment observation system 5 is arranged at the pupil conjugate position Q.

The refractometry projection system 6 and the refractometry light receiving system 7 constitute objective measurement optical systems used for objective refraction measurement (measurement) that objectively measure a refraction value as eye characteristics of the left subject eye EL. Hereinafter, the refractometry projection system 6 and the refractometry light receiving system 7 are referred to as the objective measurement optical systems 6 and 7. The refractometry projection system 6 projects a ring-shaped luminous flux (infrared light) for objective measurement from the refractometry light source 61 onto the fundus ELf. The refractometry light receiving system 7 receives the return light of the ring-shaped luminous flux from the left subject eye EL.

The refractometry light source 61 may be fabricated with a super luminescent diode (SLD) light source, which is a high luminance light source with a light emission diameter less than a predetermined size. The refractometry light source 61 is configured to move in the optical axis direction together with the focusing lens 45 and the focusing lens 74 and is disposed at the fundus conjugate position P. A ring aperture 65 (specifically, the light transmitting portion) is disposed at the pupil conjugate position Q. The focusing lens 74 is configured to move in the optical axis direction together with the refractometry light source 61 and the focusing lens 45. The focusing lens 74 may be fabricated with a known variable focus lens, which can change the focal position under the control of the controller 140. The imaging surface of the imaging element 59 in the optical system of the refractometry light receiving system 7 is disposed at the fundus conjugate position P.

The light projected from the refractometry light source 61 passes through a relay lens 62 and enters onto the conical surface of a conical prism 63. The light incident onto the conical surface is deflected and emitted from the bottom surface of the conical prism 63. The light emitted from the bottom surface of the conical prism 63 passes through a field lens 64 and passes through a light transmitting portion formed in a ring shape in the ring aperture 65. The light (ring-shaped luminous flux) passing through the light transmitting portion of the ring aperture 65 reflects on the reflection surface of an aperture prism 66, passes through a rotary prism 67, and reflects on the dichroic mirror 68. The light reflected with the dichroic mirror 68 reflets on the dichroic mirror 53, passes through the objective lens 52, and is projected onto the left subject eye EL.

The conical prism 63 is disposed at a position as close as possible to the pupil conjugate position Q desirably. The conical prism 63 may have the ring aperture 65 attached to its bottom surface facing at the field lens 64. In this case, for example, the bottom surface of the conical prism 63 may be deposited with a light shielding film so as to form a ring-shaped light transmitting portion. Alternatively, the ring aperture 65 may be on the conical surface side of the conical prism 63.

The field lens 64 has a lens surface on the side of the left subject eye EL. For example, the ring aperture 65 may be attached to the lens surface of the field lens 64. In this case, for example, the lens surface of the field lens 64 may be deposited with a light shielding film so as to form a ring-shaped light transmitting portion. Note that the refractometry projection system 6 may also be configured without the field lens 64. The ring aperture 65 may be formed with the light transmitting portion having a shape corresponding to a predetermined measurement pattern, at a position eccentric to (offset from) the optical axis of the refractometry projection system 6. The number of the light transmitting portions may be one or more than two. The rotary prism 67 is used for averaging the light amount distribution of the ring-shaped luminous flux with respect to the blood vessel and the disease site of the fundus ELf and reducing speckle noise caused by the light source.

The return light of the ring-shaped luminous flux projected on the fundus ELf passes through the objective lens 52 and reflects on the dichroic mirror 53 and the dichroic mirror 68. The return light reflected with the dichroic mirror 68 passes through the rotary prism 67, passes through the hole portion of the aperture prism 66, passes through a relay lens 71, reflects on a reflection mirror 72, and passes through a relay lens 73 and the focusing lens 74. The light passing through the focusing lens 74 reflects on a reflection mirror 75, reflects on the dichroic mirror 57, and forms an image on the imaging surface of the imaging element 59 by using the image forming lens 58.

The controller 140 calculates the parameters of the eye refraction power by a known calculation based on the output from the imaging element 59. Note that the parameters of the eye refraction power include the refraction values (refractive power), the spherical power, the astigmatism power, and the astigmatism axis angle of the left and right subject eyes EL and ER. Based on the control signal transmitted from the examiner's control device 130, the controller 140 integrally controls each portion of the measurement unit 120, which includes the drive portion 122a and the left and right measurement optical systems 125L, 125R, each of which includes the refractometry projection system 6, the refractometry light receiving system 7, the visual target projection system 4. In addition, the controller 140 transmits the measurement results regarding the eye characteristics of the left and right subject eyes EL, ER, which have been measured by using the measurement head 122, to the examiner's control device 130.

[Configuration of Controller (FIG. 3)] FIG. 3 describes the configuration of the controller 140. As illustrated in FIG. 3, the controller 140 includes a main controller 141, a storage 142, and an adaptation check portion 143.

The main controller 141 controls the light-amount accommodation and the on-and-off switching for various light sources including the Z-alignment light source 11 of the Z-alignment system 1, the XY-alignment light source 21 of the XY-alignment system 2, and the keratometric ring light source 32 of the keratometry system 3. The main controller 141 controls on/off of the visual target, which is displayed on the display 41 of the visual target projection system 4 and changing of the visual target. The main controller 141 controls the light-amount accommodation and the on-and-off switching for the anterior segment illumination light source 51 of the anterior segment observation system 5. The main controller 141 controls the exposure time and detection sensitivity adjustment for the imaging element 59 of the anterior segment observation system 5. The main controller 141 controls light-amount adjustment and the on-and-off switching for the refractometry light source 61 of the refractometry projection system 6. The main controller 141 controls rotation speed adjustment for the rotary prism 67 of the refractometry projection system 6 and the on-and-off switching for the rotation operation. In addition, the main controller 141 controls movement to move the focusing lens 45 of the visual target projection system 4, the refractometry light source 61 of the refractometry projection system 6, and the focusing lens 74 of the refractometry light receiving system 7 in the optical axis direction in an associated manner. Note that the main controller 141 reads and writes data from and into the storage 142.

The storage 142 stores various data. The data stored in the storage 142 include the measurement information acquired with the keratometry system 3, the measurement information acquired with the objective measurement optical systems 6 and 7, the image data acquired with the imaging element 59, and the subject eye information. The subject eye information includes information regarding the subject such as an ID and a name, and information regarding the left and right subject eyes EL and ER such as identification information of the left subject eye and the right subject eye. The storage 142 stores the measurement information acquired with the keratometry system 3 when the ophthalmologic apparatus 100 performs the keratometry measurement of the left and right subject eyes EL and ER. The storage 142 stores the measurement information acquired with the objective measurement optical systems 6 and 7 when the ophthalmologic apparatus 100 performs the refractometry measurement of the left and right subject eyes. The storage 142 may be used as a work memory of a calculation process of a cornea shape parameter and a calculation process of a refraction value of the left and right subject eyes EL and ER. The storage 142 stores various programs and data for operating the ophthalmic apparatus 100.

The adaptation check portion 143 accesses the adaptation abilities to stereoscopic vision of the left and right subject eye EL and ER. The adaptation check portion 143 includes a subjective examination processing portion 144, an objective measurement portion 145, and a determination portion 146.

The subjective examination processing portion 144 assumes the left and right subject eyes EL and ER as a state in which the complete correction value in the subjective examination is prescribed and presents the visual target for the left eye and the visual target for the right eye at visual target positions separated from the left and right subject eyes EL and ER by a certain examination distance (for example, 1 m), respectively. The subjective examination processing portion 144 sets the visual target for the left eye and the visual target for the right eye on the display 41 (visual target presentation portion) that presents fixed visual targets to the left and right subject eyes EL and ER included in the visual target projection system 4. The visual target for the left eye and the visual target for the right eye, which projected onto the left and right subject eyes EL and ER respectively, have the same pattern in the center of a visual target frame.

The subjective examination processing portion 144 is configured to change a convergence distance, which is a distance from the positions of the left and right subject eyes EL, ER to the convergence position where the two lines of sight of the each eye converge each other, by controlling an angle between the lines of sight from the left and right subject eyes EL and ER toward the visual target for the left eye and the visual target for the right eye. As already described and shown in FIG. 1, the ophthalmic examination apparatus 100 includes the left and right measurement head portion 122L, 122R, which are configured to measure the left and right subject eyes EL, ER and includes the visual targets for the left and right eyes and the left and right measurement optical system 125L, 125R. Based on this configuration, the subjective examination processing portion 144 controls the angle between the lines of sight by controlling rotation angle (rotation angle control) when the left measurement head portion 122L and the right measurement head portion 122R are rotated in opposite directions about two rotation axes (Y-axis direction) passing through the left and right subject eyes EL and ER. The rotation angle is controlled with the drive portion 122a based on a command from the subjective examination processing portion 144.

The rotation angle control of the left measurement head portion 122L and the right measurement head portion 122R changes the angle of the optical axis of the visual target projection system 4 from the left and right subject eyes EL and ER toward the display 41 that displays the visual target. That is, the rotation angle control is equivalent to a configuration in which a prism lens is added to any position on the optical axis of the visual target projection system 4, and the deflection angle is changed with the prism lens. Therefore, the angle between the lines of sight is hereinafter referred to as a "prism angle" indicating the prism power of the prism lens. When the light passing through the prism lens shifts by 1 cm on a vertical screen 1 m ahead, this prism power is referred to as 1 Δ (1 prism diopter), and the deflection angle at this time is 0.57 degrees. Here, the following description assumes that the prism power in the direction in which the convergence distance is close to the subject eye (the eye is rotated inward) is positive, and the prism power in the direction in which the convergence distance is far from the subject eye (the eye is rotated outward) is negative.

The subjective examination processing portion 144 is configured to control the prism angles from the left and right subject eyes EL and ER toward the target for the left eye and the visual target for the right eye in a stepwise manner, so as to change the convergence distance in a stepwise manner in a distance range from a position farther than the examination distance to a position closer than the examination distance. When the left and right subject eyes EL and ER attempt the fusion with respect to the stepwise change in the convergence distance, the subjective examination processing portion 144 acquires a subjective response from the subject.

The objective measurement portion 145 executes processes in parallel with the processes in the subjective examination processing portion 144. When the left and right subject eyes EL and ER attempt fusion with respect to a change in the convergence distance, the objective measurement portion 145 measures the eye characteristics of the left and right subject eyes EL and ER with the objective measurement optical systems 6 and 7 to acquire an objective refraction value as the objective measurement information. Here, a process in the subjective examination processing portion 144 is a process of changing the convergence distance in a stepwise manner.
Therefore, the objective measurement portion 145 measures the eye characteristics of the left and right subject eyes EL and ER a predetermined number of times in each step of the changed convergence distance and averages objective refraction values obtained a predetermined number of times in each step to acquire an average objective refraction value as the objective measurement information.

The determination portion 146 determines the adaptation abilities of the left and right subject eyes EL and ER with respect to stereoscopic vision based on the subjective response from the subject acquired with the subjective examination processing portion 144 and the objective refraction value (refractometry value) acquired with the objective measurement portion 145. When the subject's responses of the subjective examination are positive regardless of the change of the convergence distance, and the amount of the objective refraction value regardless of the change of the convergence distance is suppressed in a predetermined range, the determination portion 146 determines that the left and right subject eyes EL and ER have high adaptation ability to stereoscopic vision. When the subject's responses of the subjective examination are negative if the convergence distance is changed, and the amount of the objective refraction value if the convergence distance is changed exceeds a predetermined range, the determination portion 146 determines the left and right subject eyes EL and ER have low adaptation ability to stereoscopic vision.

[Procedure of Evaluating Adaptation Abilities to Stereoscopic Vision (FIG. 4)] The configuration and flow of actions of the process of evaluating the adaptation abilities to stereoscopic vision by using the adaptation check portion 143 are described as follows with reference to the flowchart shown in FIG. 4. FIG. 4 illustrates an example of the operation of the adaptation check portion 143.

Step S1 is a preliminary measurement step in which the main controller 141 acquires the eye characteristics including the refraction values of the left and right subject eyes EL and ER based on the captured image of the ring-shaped image or the like. Note that the main controller 141 may execute the preliminary measurement twice or more to determine the measurement conditions of the same type or those of different types by executing each of the preliminary measurement so that the measurement conditions should be converged each time while the preliminary measurement is repeated. For example, preliminary measurement each time determines, based on the obtained ring-shaped image, at least one of the light amount from the refractometry light source 61, the exposure time of the imaging element 59, the detection sensitivity of the imaging element 59, and the control content for the focusing lens 74.

Step S2 is a main measurement step in which the main controller 141 measures the objective refraction values of the target left and right subject eyes EL and ER under the measurement conditions determined by preliminary measurement executed the last time. Note that the objective refraction values of the left and right subject eyes EL and ER are measured by the following sequence. That is, the main controller 141 projects a ring-shaped luminous flux (infrared light) for objective measurement onto the fundi ELf and ERf of the left and right subject eyes EL and ER with the refractometry projection system 6. Then, the main controller 141 detects (receives an image of) the return light of the ring-shaped luminous flux from the fundi ELf and ERf with the refractometry light receiving system 7 and acquires the objective refraction values of the left and right subject eyes EL and ER by a known method based on the image signal from the imaging element 59.

Step S3 is a start setting step of the subjective examination in which the main controller 141 sets the start of the subjective examination for each of the left and right subject eyes EL and ER. Here, the "start setting of the subjective examination" means that the position of the focusing lens 45 in the visual target projection system 4 is adjusted based on the refraction values of the left and right subject eyes EL and ER obtained in the main measurement such that the spherical power of the left and right subject eyes EL and ER is in the complete correction state at the examination distance at the distant position.

Step S4 is an RG test step in which the main controller 141 performs an RG test using an RG chart. Here, the "RG test" refers to an examination for subjectively checking whether the correction state of each of the left and right subject eyes EL and ER is in a complete correction state (proper) (whether the correction is not overcorrection or undercorrection) using a characteristic of light referred to as chromatic aberration using the RG chart. In addition, in the "RG chart", for example, red icons having a number with a different size and a double circle and a circle with a different size as visual targets and green icons with visual targets having the same shape as the red icons are arranged side by side. In the RG test, due to the characteristics of the light transmissive body of the eye, as the wavelength of light is shorter, the power on the refractive surface is larger. Therefore, the green wavelength light forms an image at a position shifted in the incident side direction as compared with the red wavelength light. Therefore, when the left and right subject eyes EL and ER are undercorrected, the red visual target is more clearly viewed, and when the left and right subject eyes EL and ER are overcorrected, the green visual target is more clearly viewed. Therefore, step S4 and step S5 are repeated until the green and red visual targets of the RG chart are equally viewed, and the subject adjusts the spherical power (correction degree) of the left and right subject eyes EL and ER by changing the position of the focusing lens 45. As a result, the left and right subject eyes EL and ER are prescribed by the complete correction value based on a provisional determination value in the RG test.

Note that steps S1 to S4 correspond to a complete correction prescription step of assuming the left and right subject eyes EL and ER as a state where a complete correction value in the subjective examination is prescribed. However, the complete correction values of the left and right subject eyes EL and ER may not be provisional determination values in the RG test in Example 1, but may be any value of, for example, a value separately measured, a prescription value in the current eyeglasses, a value determined by performing the subjective optometry in a general way, for example. In addition, at this time, the eye positions of the subject eyes EL and ER may be corrected by an examination for heterophoria or strabismus.

Step S5 is a visual target presentation step in which the subjective examination processing portion 144 sets an initial prism angle and the examination distance to match a convergence distance to a convergence position when the visual target for the left eye and the visual target for the right eye are fused, with the examination distances from the left and right subject eyes EL and ER to the visual target positions. For example, the subjective examination processing portion 144 controls rotation on the initial prism angle so that the convergence distance and the examination distance match (0 Δ), and controls the focusing lens to set the examination distance from the left and right subject eyes EL and ER to a position of 1 m. This step S5 corresponds to the visual target presentation step of presenting the visual target for the left eye and the visual target for the right eye having the same pattern in the center position of the visual target frame on the display 41 (visual target presentation portion) to the visual target position separated from the left and right subject eyes EL and ER by a certain examination distance, subsequent to the prescription of the complete correction value.

Step S6 is a subjective response step in which the subjective examination processing portion 144 acquires a subjective response regarding fusion of the visual target for the left eye and the visual target for the right eye from the subject. The subjective examination processing portion 144, as the subjective response, acquires, for example, a response as to whether the visual targets are seen as one visual target by fusion of the visual target for the left eye and the visual target for the right eye. Here, when the visual targets are not seen as one visual target, it is checked whether the subject eye can be appropriately examined. Then, when the eye cannot be examined, setting is performed again so as to be in an appropriate state, and when the eye can be examined, examination may be stopped, or examination may be continued with eye positions corrected so that the visual targets are seen as one visual target.

Step S7 is a prism angle change processing step in which the subjective examination processing portion 144 controls rotation angle of the drive portion 122a to change the prism angle from 0 Δ or a prism angle of the previous time to the next prism angle in a stepwise manner with the examination distance (for example, 1 m) maintained. The prism angle is changed in 11 steps of, for example, 0 Δ, ±1 Δ, ±2 Δ, ±4 Δ, ±6 Δ, and ±10 Δ. For example, when the initial prism angle is 0 Δ, the prism angle is changed in 11 steps from +1 Δ to +2 Δ to +4 Δ to +6 Δ to +10 Δ to -1 Δ to -2 Δ to -4 Δ to -6 Δ, and to -10 Δ. Note that, this example examines in a direction from a position where the examination distance and the convergence distance match with each other to a direction approaching the subject eye. Then, after the distance is changed to a predetermined distance, the position is returned to the position where the examination distance and the convergence distance match with each other, and then examines in a direction separating from the position. However, the present disclosure is not limited thereto, and this example may examine in the approaching direction after the examining in the separating direction first. In addition, when the approaching direction and the separating direction are switched, the subjective examination processing portion 144 may perform the examination again, when the position is returned to the position where the examination distance and the convergence distance match with each other and continue the examination.

Step S8 is a subjective response step in which the subjective examination processing portion 144 acquires a subjective response related to fusion of the visual target for the left eye and the visual target for the right eye from the subject, when the fusion is attempted with the left and right subject eyes EL and ER with respect to the stepwise change in the convergence distance. The subjective examination processing portion 144, as the subjective response, acquires, for example, a response as to whether the visual targets are seen as one visual target by fusion.

Step S9 is a final prism angle determination step in which the subjective examination processing portion 144 determines whether the prism angle is changed to the final prism angle (for example, -10 Δ). While the subjective examination processing portion 144 determines NO in step S9, the process returns to step S7, and when the subjective examination processing portion 144 determines YES in step S9, the process proceeds to step S13.

Steps S7 and S9 correspond to the convergence distance control step of prescribing a state in which the convergence distance from the positions of the left and right subject eyes EL and ER to the convergence position where the two lines of sight intersect each other changes by control to change a prism angle from the left and right subject eyes EL and ER toward the visual target for the left eye and the visual target for the right eye. Steps S6 and S8 correspond to a subjective response acquisition step of acquiring a subjective response related to fusion from the subject when fusion is attempted with the left and right subject eyes EL and ER with respect to a change in the convergence distance.

Step S10 is a refractometry step in which the objective measurement portion 145 measures the eye characteristics of the left and right subject eyes EL and ER with respect to the change in the convergence distance with the objective measurement optical system and acquires the objective refraction value as the objective measurement information. Step S11 is a refractometry value display step in which the objective measurement portion 145 displays a plurality of objective refraction values (refractometry values) acquired at the timing when the prism angle changes. Step S12 is an average refractometry value calculation step in which the objective measurement portion 145 compares a plurality of refractometry values acquired at the timing when the prism angle changes and calculates an average value. These steps S10 to S12 correspond to the accommodation reaction amount measurement step of being executed in parallel with the control of the convergence distance, measuring the eye characteristics of the left and right subject eyes EL and ER with the objective measurement optical system, when fusion is attempted with the left and right subject eyes EL and ER to the change of the convergence distance, and acquiring the objective refraction value as the objective measurement information.

Step S13 is a step in which the determination portion 146 determines adaptation abilities of the left and right subject eyes EL and ER to the stereoscopic vision based on the subjective response from the subjective examination processing portion 144 and the objective refraction value (average refractometry value) from the objective measurement portion 145. The process proceeds to the end, when the determination of the adaptation abilities of the stereoscopic vision is completed in step S13. This step S13 corresponds to a determination step of determining the adaptation abilities of the left and right subject eyes EL and ER to the stereoscopic vision based on the subjective response and the objective refraction value.

The flow of actions of each step is described as follows. The process of FIG. 4 starts when alignment in which the subject faces the ophthalmic examination apparatus 100 and sets the face at a predetermined position, and appropriately adjusts the position relationship between the left and right subject eyes EL and ER and the apparatus is completed, and the process proceeds from step S1 to step S2 to step S3 and to step S4. After starting, the main controller 141 performs preliminary measurement (step S1), main measurement (step S2), and start setting of subjective examination (step S3). In step S4, after the start setting of the subjective examination, the main controller 141 performs the RG test using the RG chart, thereby assuming the left and right subject eyes EL and ER as a state in which the complete correction value is prescribed by the provisional determination value in the RG test.

The process of FIG. 4 proceeds from step S5 to step S6 after the complete correction value is in a state of being prescribed for the left and right subject eyes EL and ER in step S4. In step S5, the subjective examination processing portion 144 controls the rotation angle of the drive portion 122a with the prism angle as the initial prism angle and movement control of the focusing lens 45 with the examination distance set to a constant distance. In step S6, the subjective examination processing portion 144 acquires, from the subject, a subjective response related to fusion when the convergence distance and the examination distance are matched.

The process in FIG. 4 proceeds from step S7 to step S8 to step S9 after the subjective examination processing portion 144 acquires the subjective response related to the fusion in the initial setting from the subject. In step S7, the subjective examination processing portion 144 controls the rotation angle of the drive portion 122a that switches the prism angle from the initial prism angle (for example, 0 Δ) to the prism angle in which the convergence distance becomes a predetermine convergence distance. In step S8, the subjective examination processing portion 144 acquires, from the subject, the subjective response related to the fusion when the prism angle is switched to the prism angle in which the convergence distance becomes the predetermined convergence distance. In step S9, the subjective examination processing portion 144 determines whether the convergence distance is changed by all the predetermined prism angles.

In the process of FIG. 4, in step S9, while the subjective examination processing portion 144 determines that the prism angle is not changed to the final prism angle (for example, -10 Δ), the flow from step S7 to step S8 to step S9 is repeated. In step S7, the subjective examination processing portion 144 controls the rotation angle of the drive portion 122a to switch the prism angle from the current prism angle to the prism angle of the next step. The prism angle is switched in a stepwise manner like from 0 Δ to + 1 Δ, from +1 Δ to +2 Δ, from +2 Δ to +4 Δ, from +4 Δ to +6 Δ, from +6 Δ to +10 Δ, from +10 Δ to -1 Δ, from -1 Δ to -2 Δ, from -2 Δ to -4 Δ, from -4 Δ to - 6 Δ, and from -6 Δ to -10 Δ. In step S8, the subjective examination processing portion 144 acquires, from the subject, the subjective response related to the fusion in each step of the switched prism angle.

In the process of FIG. 4, in step S5, when the subjective examination processing portion 144 controls the matching of the convergence distance with the examination distance, the objective measurement portion 145 starts the objective measurement in parallel with the subjective examination process. For the objective measurement, the subjective examination process repeats the flow proceeding to step S10 to step S11 to step S12. When the subjective examination process ends (YES in step S9), the objective measurement ends in accordance with the end of the subjective examination process. In step S10, the objective measurement portion 145 acquires the objective refraction value (refractometry value) as the objective measurement information by the refractometry. In step S11, the objective measurement portion 145 displays a plurality of objective refraction values (refractometry values) acquired at the timing when the prism angle changes. In step S12, the objective measurement portion 145 compares a plurality of refractometry values acquired at the timing when the prism angle changes to calculate the average value.

In the process of FIG. 4, in step S9, while the subjective examination processing portion 144 determines that the prism angle is changed to the final prism angle (for example, -10 Δ), the process proceeds from step S9 to step S13. In step S13, the determination portion 146 determines adaptation abilities of the left and right subject eyes EL and ER to the stereoscopic vision based on the subjective response from the subjective examination processing portion 144 and the objective refraction values (average refractometry values) from the objective measurement portion 145.

As described above, the flow of the processing action of evaluating the adaptation abilities to the stereoscopic vision is executed in parallel with the subjective examination process and the objective measurement and merges with the objective measurement in step S13 when the subjective examination process ends. In step S13, for example, when a subjective response that the fusion by binocular vision is achieved is obtained irrelevant to the stepwise change of the convergence distance and the change of the average refractometry value is suppressed in a predetermined range, the determination portion 146 determines that the left and right subject eyes EL and ER have high adaptation abilities to stereoscopic vision. In step S13, for example, by changing the convergence distance in a stepwise manner, when the subjective response that two visual targets are seen is obtained and fusion by binocular vision is not formed, or the change of the average refractometry value exceeds the predetermined range, the determination portion 146 determines that the left and right subject eyes EL and ER have low adaptation abilities stereoscopic vision.

[Visual Target for Left Eye and Visual Target for Right Eye (FIGS. 5A and 5B)] FIGS. 5A and 5B describe a visual target for the left eye 8L and a visual target for the right eye 8R set on the display 41 (visual target presentation portion).

The visual target for the left eye 8L is a visual mark projected on the left subject eye EL when the adaptation check portion 14 evaluates the adaptation abilities of stereoscopic vision. As illustrated in FIG. 5A, the visual target for the left eye 8L has an asterisk mark 82 (pattern) at the center position of a rectangular visual mark frame 81 such as a liquid crystal frame.

The visual target for the right eye 8R is a visual target projected on the right subject eye ER when the adaptation check portion 143 evaluates the adaptation abilities of stereoscopic vision. As illustrated in FIG. 5B, the visual target for the right eye 8R has the asterisk mark 82 (pattern) at the center position of the rectangular visual target frame 81 such as a liquid crystal frame.

As is clear from the comparison between FIGS. 5A and 5B, the visual target for the left eye 8L and the visual target for the right eye 8R have the asterisk marks 82 with the same pattern, the same size, and the same line segment width at the center position of the visual target frame 81. A pattern, a size, and a line segment width of the asterisk mark 82 are determined so as to appropriately provide fusion stimulation to the left and right subject eyes EL and ER at the time of fusion in which images appearing on the retina at the time of binocular vision are combined into one and viewed as a single image.

Note that, when the visual target frame 81 is a liquid crystal frame, the visual target frame 81 has weak fusion stimulation, and thus fusion stimulation can be increased by using a black frame with the thick line width. Furthermore, the variable line width of the brake frame in the visual target frame 81 allows the variable strength of the fusion stimulation. In addition, changing the size or the radial line segment width of the asterisk mark 82 allows the variable strength of the fusion stimulation. Furthermore, the asterisk mark 82 can vary the strength of fusion stimulation by changing the visual target pattern to another pattern.

[Relationship Between Examination Distance and Convergence Distance (FIG. 6)] The subjective examination processing portion 144 prescribes a state in which the convergence distance from the positions of the left and right subject eyes EL and ER to the convergence position where the two lines of sight intersect changes by control to change a prism angle from the left and right subject eyes EL and ER toward the visual target for the left eye 8L and the visual target for the right eye 8R. FIG. 6 describes the relationship between the convergence distance and the examination distance.

As illustrated in FIG. 6, an "examination distance Lid" refers to a distance from the left and right subject eyes EL and ER to a position where the visual target presents in the Z-axis direction. The visual target projection system 4 that presents the visual mark on the display 41 obtains the examination distance Lid. In addition, the examination distance Lid can be referred to as a focus accommodation distance when the visual target is focused with the left and right subject eyes EL and ER. Here, the examination distance Lid is the distance of the visual target from the straight line connecting the left and right subject eyes EL and ER, but is not limited thereto, and may be the distance of the visual target from each of the eyes EL and ER.

The convergence distance refers to a distance in the Z-axis direction from the positions of the left and right subject eyes EL and ER to a convergence position where two lines of sight intersect each other. As illustrated in FIG. 6, a convergence distance L2 (= examination distance Lid) is a distance in the Z-axis direction from the positions of the left and right subject eyes EL and ER to a convergence position R2 where two lines of sight SL2 and SR2 intersect each other. Also, as illustrated in FIG. 6, a convergence distance L1 is a distance in the Z-axis direction from the positions of the left and right subject eyes EL and ER to a convergence position R1 where two lines of sight SL1 and SR1 intersect each other.

Here, the examination distance is maintained at the examination distance Lid, and the convergence distance is changed from the convergence distance L2 to the convergence distance L1 as indicated by an arrow A. At this time, with the change of the convergence distance from the convergence distance L2 to the convergence distance L1, the convergence angle is changed from a convergence angle Θ2 at which the lines of sight SL2 and SR2 intersect each other to a convergence angle θ1 (> θ2) at which the lines of sight SL1 and SR1 intersect each other. Therefore, as the convergence angle is changed from the convergence angle θ2 to the convergence angle θ1, the left and right subject eyes EL and ER move in a direction in which the left and right subject eyes EL and ER approach, and the eye movements become convergence stimulation.

Meanwhile, since the examination distance is maintained at the examination distance Lid, the focus accommodation distance with the left and right subject eyes EL and ER is also constant. Therefore, since the focus accommodation distance of the left and right subject eyes EL and ER is maintained constant, even if the convergence distance is changed from the convergence distance L2 to the convergence distance L1, the accommodation function by the change of the examination distance Lid fails to work. Therefore, no objective refraction value changes in principle when the left and right subject eyes EL and ER are objectively measured. However, when the accommodation function that is induced by the convergence stimulation and changes the curvature of the lens works on the left and right subject eyes EL and ER, the objective refraction values change when the left and right subject eyes EL and ER are objectively measured.

As described above, the relationship in which the examination distance is constant, and the convergence distance changes is a relationship in which only convergence stimulation is given to the left and right subject eyes EL and ER. Therefore, the adaptation check portion 143 of Example 1 has a configuration in which, when two visual targets are arranged at positions at a certain distance from the left and right subject eyes EL and ER, and the two visual targets are fused with both eyes, a state in which convergence stimulation is given by a change in prism angle, and a discrepancy occurs between convergence and accommodation is reproduced. For this reason, the adaptation check portion 143 can evaluate whether the left and right subject eyes EL and ER have high adaptation abilities to stereoscopic vision in which a discrepancy between convergence and accommodation occurs.

[Stepwise Changing Action of Convergence Distance (FIGS. 7Ato 7C)] The subjective examination processing portion 144 prescribes a state in which a convergence position R (±n Δ) and a convergence distance L (±n Δ) change in a stepwise manner in a distance range from a position farther than the examination distance Lid to a position closer than the examination distance Lid by control to change the prism angle in a stepwise manner. FIGS. 7Ato 7C describes the changing action of the convergence distance L (±n Δ).

FIG. 7A illustrates a state in which the convergence distance L (0 Δ) to the convergence position R (0 Δ) when the visual target for the left eye 8L and the visual target for the right eye 8R are fused is matched with the examination distance Lid from the left and right subject eyes EL and ER to the visual target position (step S5). FIG. 7B illustrates a state in which the convergence distance L (+k Δ) to the convergence position R (+k Δ) when the visual target for the left eye 8L and the visual target for the right eye 8R are fused is closer than the examination distance Lid from the left and right subject eyes EL and ER to the visual target position. Note that (+k Δ) is any one of (+2 Δ) to (+10 Δ). FIG. 7C illustrates a state in which the convergence distance L (-k Δ) to the convergence position R (-k Δ) when the visual target for the left eye 8L and the visual target for the right eye 8R are fused is farther than the examination distance Lid from the left and right subject eyes EL and ER to the visual target position. Note that (-k Δ) is any one of (-2 Δ) to (-10 Δ).

In step S5 of FIG. 4, the subjective examination processing portion 144 sets the examination distance Lid to 1 m and sets the prism angle to the initial prism angle (0 Δ). Therefore, as illustrated in FIG. 7A, the convergence distance L (0 Δ) to the convergence position R (0 Δ) when the two visual targets are fused with the left and right subject eyes EL and ER matches with the examination distance Lid from the left and right subject eyes EL and ER to the visual target position.

In step S7 of FIG. 4, the subjective examination processing portion 144 controls the prism angle in 11 stages of 0 Δ, ±1 Δ, ±2 Δ, ±4 Δ, ±6 Δ, and ±10 Δ while maintaining the examination distance Lid (for example, 1 m). Therefore, as illustrated in FIG. 7C, when the prism angle is -k Δ, the convergence distance L (-k Δ) to the convergence position R (-k Δ) when the two visual targets are fused with the left and right subject eyes EL and ER is farther than the examination distance Lid from the left and right subject eyes EL and ER to the visual target position. Meanwhile, as illustrated in FIG. 7B, when the prism angle is +k Δ, the convergence distance L (+k Δ) to the convergence position R (+k Δ) when the two visual targets are fused with the left and right subject eyes EL and ER is closer than the examination distance Lid from the left and right subject eyes EL and ER to the visual target position.

When the subjective examination processing portion 144 controls the prism angle in 11 steps in this manner, the state is changed in a stepwise manner from the state in which the convergence distance L (-k Δ) approaches the examination distance Lid (FIG. 7C) to the state in which the convergence distance L (-k Δ) is separated from the examination distance Lid (FIG. 7B). Therefore, by changing the convergence distance L (±n Δ) from a state of being farther than the examination distance Lid to a state of being closer than the examination distance Lid in a stepwise manner, the subjective examination processing portion 144 can give the convergence stimulation that changes in a stepwise manner to the left and right subject eyes EL and ER.

[Measurement of Temporal Change of Accommodation and Convergence during Stereoscopic Image Observation (FIGS. 8A to 8E)] The present inventors measured the temporal change of the accommodation and the convergence separately for eyes of a healthy group and eyes of a stereoscopic image fatigue group. Hereinafter, FIGS. 8A to 8E describe the measurement of the temporal change of the accommodation and convergence.

The prism angle of the visual target is changed to a position where the examination distance is 60 cm, and the parallax distance is 4 cm (6.7 Δ, amount of protrusion at this time is 24 cm). FIG. 8A illustrates the measurement conditions as follows: the parallax of 0 degree is maintained from one to two seconds, the prism angle is changed from 0 Δ to 6.7 Δ over two seconds, and then the prism angle of 6.7 Δ is fixed.

FIG. 8B shows the measurement results by the healthy eye example. When the prism angle is changed from 0 Δ to 6.7 Δ over 2 seconds, the value of the convergence (MA) decreases following the change in the prism angle. When the prism angle of 6.7 Δ is fixed, the value of the convergence (MA) slightly fluctuates, but the value due to the protruding position after the decrease is maintained. Note that "Meter Angle (MA)" is a unit of convergence force. When the prism angle is changed from 0 Δ to 6.7 Δ over 2 seconds, the focus accommodation function for the protruding position works to decrease the value of the refractivity (D). However, when the prism angle of 6.7 Δ is fixed, the decreased value of the refractivity (D) returns to the original value with a good response, and then the original value is maintained. Note that "Diopter (D)" is a unit of the refractivity in which the refractive power is replaced with a number.

FIGS. 8D and 8E show the measurement results of the stereoscopic image fatigue group. The measurement results of the convergence (MA) and the refractivity (D) in FIG. 8D are an example in which a user feels a headache and eye fatigue at the time of viewing an IMAX movie ("IMAX" is a registered trademark) for about 15 minutes and experiences discontinuation of viewing. Here, the "IMAX movie" refers to a movie based on a high-definition video shown with a special projection system using a moving image film of a special standard (a standard in which the area of a film used for one frame is widened from a normal 35 mm to 70 mm). Irrelevant to the change or maintenance of the prism angle, both the convergence (MA) and the refractivity (D) show disturbed characteristics such as including fluctuation of a large value and interruption of a value in the middle.

The measurement results of the convergence (MA) and the refractivity (D) in FIG. 8E are an example in which a user feels strong sleepiness and eye fatigue at the time of viewing a 3D movie for about 30 minutes and experiences discontinuation of viewing. Here, the "3D movie" refers to a movie in which a video projected on a screen is stereoscopically shown with special glasses or the like. When the prism angle is changed from 0 Δ to 6.7 Δ over 2 seconds, the value of the convergence (MA) at the time of start of the change is lower than that in case of the healthy eye example but decreases following the change in the prism angle. When the prism angle of 6.7Δ is fixed, the value of the convergence (MA) depending on the protruding position tends to be maintained, but the value is interrupted in the middle, which indicates a disturbed characteristic. When the prism angle is changed from 0 Δ to 6.7 Δ over 2 seconds, the focus accommodation function for the protruding position works to decrease the value of the refractivity (D). When the prism angle of 6.7 Δ is fixed, the refractivity (D) shows a disturbed characteristic due to the fluctuation of a value for returning a decreased value to an original value or a value interruption in the middle.

FIG. 8E shows the analysis result examples of the temporal change frequency of the refractivity (D) by the healthy group and the fatigue group. When the magnitude of the amplitude is compared between the healthy group and the fatigue group, the amplitude (fluctuation range) is larger in the fatigue group than in the healthy group in any of the frequency bands of 0.5 to 1.5 Hz, 1.5 to 2.5 Hz, 2.5 to 3.5 Hz, and 3.5 to 4.5 Hz. Therefore, the analysis result example illustrated in FIG. 8E can be used as data for checking that the subject eye belongs to the fatigue group with the magnitude of the fluctuation range when the amplitude (fluctuation range) of the temporal change frequency of the refractivity (D) of the subject eye is measured. In particular, in a low frequency band, the analysis result example can be used as the data for checking that the subject eye belongs to the fatigue group with the magnitude of the amplitude.

As illustrated in FIG. 8E, for example, in a low frequency band of 0.5 to 1.5 Hz, the determination threshold for determining that the subject eye belongs to the fatigue group is set to about an amplitude of 0.2 to 0.3. In this case, when the stereoscopic adaptation evaluation examination is performed on a predetermined subject eye, and the objective refraction value data is acquired in time series along the time axis, the amplitude in the low frequency band of 0.5 to 1.5 Hz of the objective refraction value characteristic by the data is measured. Then, when the measured amplitude of the low frequency band is a preset determination threshold or more, the subject eye can be determined as belonging to the fatigue group. Note that, here, the examination is performed by changing the prism angle with the internal visual target, but the present disclosure is not limited thereto. For example, a similar examination can be performed by installing a naked-eye stereoscopic display and presenting an image in which the line-of-sight angle is changed to each of the left and right subject eyes.

[Action of determining adaptation ability to Stereoscopic Vision (FIG. 9)] The objective measurement portion 145 measures the eye characteristics of the left and right subject eyes EL and ER a predetermined number of times in each step of the convergence distance L (±n Δ) and acquires an average objective refraction value obtained by averaging the objective refraction values obtained a predetermined number of times in each step as the objective measurement information. FIG. 9 describes the action of determining the adaptation ability to stereoscopic vision.

FIG. 9 illustrates a relationship characteristic between the prism angle and the objective refraction value when the average objective refraction value is acquired in each step of the convergence distance L (±n Δ) for a plurality of subjects. The vertical axis represents an objective refraction value, and the unit of each numerical value is a diopter (D). The horizontal axis represents a prism angle, and the unit of each numerical value is a prism (Δ) but may be converted into a diopter (D) or a convergence angle (MA) represented by a reciprocal number of a convergence distance.

A first group G1 of the relationship characteristics in FIG. 9 shows a characteristic that the numerical value of the objective refraction value tends to rise in response to the switching of the prism angle when the prism angle is changed in seven steps of -1 Δ to 0 Δ to +1 Δ to +2 Δ to +4 Δ to +6 Δ to +10 Δ. That is, when the prism angle becomes +2 Δ to +4 Δ to +6 Δ to +10 Δ, the first group G1 has a characteristic that the numerical value of the objective refraction value rises to about D = 3.0 accordingly.

For this reason, the focus accommodation reactions of the left and right subject eyes EL and ER of the subject belonging to the first group G1 may be induced, while the subject belonging to the first group G1 receives convergence stimulation due to a change in the convergence distance. Therefore, a discrepancy between the refraction value and the visual target position occurs due to the convergence, and thus the left and right subject eyes EL and ER of the subject belonging to the first group G1 can be determined as having low adaptation abilities to stereoscopic vision.

Meanwhile, a second group G2 of the relationship characteristics in FIG. 9 shows a characteristic that the numerical value of the objective refraction value transitions in a state of crawling when the prism angle is changed in seven steps of -1 Δ to +0 Δ to +1 Δ to +2 Δ to +4 Δ to +6 Δ to +10 Δ. That is, even when the prism angle becomes +2 Δ to +4 Δ to +6 Δ to +10 Δ, the second group G2 has a characteristic that the numerical value of the objective refraction value is suppressed to about D < 1.0.

For this reason, the focus accommodation reactions of the left and right subject eyes EL and ER of the subject belonging to the second group G2 may not be induced, even if the subject belonging to the second group G2 receives convergence stimulation due to a change in the convergence distance. Therefore, a discrepancy between the refraction value and the visual target position does not occur due to the convergence, and thus the left and right subject eyes EL and ER of the subject belonging to the second group G2 can be determined as having high adaptation abilities to stereoscopic vision.

### [Effects of Ophthalmic Examination Apparatus and Ophthalmic Examination Method]

(1) The ophthalmic examination apparatus 100 includes the visual target projection system 4 including the visual target presentation portion (the displays 41) that presents fixed visual targets to the left and right subject eyes EL and ER, the objective measurement optical systems 6 and 7 that objectively measure the eye characteristics of the left and right subject eyes EL and ER, and the controller 140 that controls each portion of the apparatus. The fixed visual targets are the visual target for the left eye 8L and the visual target for the right eye 8R which are projected onto the left and right subject eyes EL and ER, respectively, and have the same pattern (asterisk mark 82). The visual target presentation portion presents the visual target for the left eye 8L and the visual target for the right eye 8R at the visual target positions separated from the left and right subject eyes EL and ER by the certain examination distance Lid. The visual target projection system 4 prescribes a state in which the convergence distance L (±n Δ) from the positions of the left and right subject eyes EL and ER to the convergence position R (±n Δ) where the two lines of sight intersect each other changes by changing an angle between the lines of sight (prism angle) from the left and right subject eyes EL and ER toward the visual target for the left eye 8L and the visual target for the right eye 8R. When fusion is attempted with the left and right subject eyes EL and ER to a change in the convergence distance L (±n Δ), the controller 140 measures the eye characteristics of the left and right subject eyes EL and ER with the objective measurement optical systems to acquire an objective refraction value as the objective measurement information. For this reason, the ophthalmic examination apparatus 100 can evaluate whether the left and right subject eyes EL and ER have high adaptation abilities to stereoscopic vision in which a discrepancy between convergence and accommodation occurs.
(2) The ophthalmic examination apparatus 100 is an objective measurement machine with a subjective examination function. The controller 140 includes the adaptation check portion 143 that evaluates the adaptation abilities with the left and right subject eyes EL and ER, and the adaptation check portion 143 includes the subjective examination processing portion 144 and the objective measurement portion 145. The subjective examination processing portion 144 presents the visual target for the left eye 8L and the visual target for the right eye 8R at the visual target positions separated from the left and right subject eyes EL and ER by the certain examination distance Lid. A state in which the convergence distance L (±n Δ) from the positions of the left and right subject eyes EL and ER to the convergence position R (±n Δ) where the two lines of sight intersect each other changes is prescribed by the control to change the angle between the lines of sight (prism angle) from the left and right subject eyes EL and ER toward the visual target for the left eye 8L and the visual target for the right eye 8R. The objective measurement portion 145 executes processes in parallel with the process in the subjective examination processing portion 144, measures the eye characteristics of the left and right subject eyes EL and ER with the objective measurement optical systems 6 and 7 when fusion is attempted with the left and right subject eyes EL and ER to a change in the convergence distance L (±n Δ), and acquires an objective refraction value as the objective measurement information. Therefore, the ophthalmic examination apparatus 100 can save an effort and time required for the subjective examination as compared with the case where the subjective examination process is performed by a manual operation. In addition, the ophthalmic examination apparatus 100 can accurately evaluate whether the left and right subject eyes EL and ER have high adaptation abilities to stereoscopic vision by executing the subjective examination process and the objective measurement by concurrent processing by simultaneous progress.
(3) The ophthalmic examination apparatus 100 includes the left measurement head portion 122L that corresponds to the left subject eye EL and is equipped with the visual target for the left eye 8L and the left measurement optical system 125L and the right measurement head portion 122R that corresponds to the right subject eye ER and is equipped with the visual target for the right eye 8R and the right measurement optical system 125R. The subjective examination processing portion 14controls the angle between the lines of sight (prism angle) by controlling a rotation angle by which the left measurement head portion 122L and the right measurement head portion 122R are rotated in opposite directions. Therefore, when the convergence distance L (±n Δ) is changed by the rotation control of the left measurement head portion 122L and the right measurement head portion 122R, the subjective examination processing portion 144 can maintain a state in which visual axes from the left and right subject eyes EL and ER is matched with the optical axes in the left measurement optical system 125L and the right measurement optical system 125R. In addition, since the visual axis and the optical axis are matched with each other, the ophthalmic examination apparatus 100 can measure eye positions and directions of the lines of sight for checking whether objective binocular vision can be performed with the left and right subject eyes EL and ER from the anterior segment image.
(4) When the fusion is attempted with the left and right subject eyes EL and ER with respect to the change in the convergence distance, the subjective examination processing portion 144 acquires a subjective response from the subject. The adaptation check portion 143 includes, in addition to the subjective examination processing portion 144 and the objective measurement portion 145, the determination portion 146 that determines the adaptation abilities of the left and right subject eyes EL and ER to stereoscopic vision based on the subjective response from the subject acquired with the subjective examination processing portion 144 and the objective refraction value acquired with the objective measurement portion 145. Therefore, when determining the adaptation abilities of the left and right subject eyes EL and ER to stereoscopic vision, the determination portion 146 can check whether fusion is subjectively achieved.
(5) When a positive subjective response to the fusion by binocular vision is obtained with respect to the change of the convergence distance L (±n Δ), and the change width of the objective refraction value is suppressed in a predetermined range, the determination portion 146 determines that the left and right subject eyes EL and ER have high adaptation abilities to stereoscopic vision. For this reason, when the fusion is subjectively generated while the convergence stimulation is given by the change in the convergence distance L (±n Δ) and the focus accommodation reaction of the left and right subject eyes EL and ER is blunt, the determination portion 146 can determine that the left and right subject eyes EL and ER have high adaptation abilities to stereoscopic vision.
(6) When a negative subjective response to the fusion by binocular vision is obtained with respect to the change of the convergence distance L (±n Δ), and the change width of the objective refraction value exceeds a predetermined range, the determination portion 146 determines that the left and right subject eyes EL and ER have low adaptation abilities to stereoscopic vision. For this reason, when the subjective fusion is difficult while the convergence stimulation is given by the change in the convergence distance L (±n Δ), and the focus accommodation reaction of the left and right subject eyes EL and ER occurs, the determination portion 146 can determine that the left and right subject eyes EL and ER have low adaptation abilities to stereoscopic vision.
(7) The adaptation check portion 143 includes, in addition to the subjective examination processing portion 144 and the objective measurement portion 145, the determination portion 146 that determines the adaptation abilities of the left and right subject eyes EL and ER to stereoscopic vision based on the objective refraction value acquired with the objective measurement portion 145. The determination portion 146 measures the amplitude which is the fluctuation range of the refraction value characteristic with the time axis of the objective refraction value acquired with the objective measurement portion 145 and determines that the left and right subject eyes EL and ER belong to the fatigue group when the fluctuation frequency of the refraction value characteristic is in the low frequency band and the amplitude is the determination threshold or more. Therefore, the determination portion 146 can determine that the left and right subject eyes EL and ER belong to the fatigue group that has low adaptation abilities to stereoscopic vision based on the refraction value characteristic with the time axis of the acquired objective refraction value.
(8) The subjective examination processing portion 144 prescribes a state in which the convergence distance L (±n Δ) changes in a stepwise manner in a distance range from a position farther than the examination distance Lid to a position closer than the examination distance Lid by control to change the angles between the lines of sight (prism angles) from the left and right subject eyes EL and ER toward the visual target for the left eye 8L and the visual target for the right eye 8R in a stepwise manner. The objective measurement portion 145 measures the eye characteristics of the left and right subject eyes EL and ER a predetermined number of times in each step of the convergence distance L (±n Δ) and acquires an average objective refraction value obtained by averaging the objective refraction values obtained a predetermined number of times in each step as the objective measurement information. Therefore, the subjective examination processing portion 144 can give convergence stimulation that changes in a stepwise manner to the left and right subject eyes EL and ER. In addition, the objective measurement portion 145 can acquire information indicating the accommodation reaction amount of the left and right subject eyes EL and ER with respect to a convergence stimulation that changes in a stepwise manner, by an average objective refraction value that is objective measurement information.
(9) The ophthalmic examination method includes the complete correction prescription step (steps S1 to S4), the visual target presentation step (step S5), the convergence distance control step (step S7 and step S9), the accommodation reaction amount measurement step (steps S10 to S12), and a determination step (step S13). The complete correction prescription step assumes the left and right subject eyes EL and ER as a state in which a complete correction value in subjective examination is prescribed. The visual target presentation step presents the visual target for the left eye 8L and the visual target for the right eye 8R having the same pattern (asterisk mark 82) on the visual mark presentation portion (display 41) to the visual target position separated from the left and right subject eyes EL and ER by the certain examination distance Lid, subsequent to the prescription of the complete correction value. The convergence distance control step prescribes a state in which the convergence distance L (0 Δ) from the positions of the left and right subject eyes EL and ER to the convergence position R (0 Δ) where the two lines of sight intersect each other changes by changing the angle between the lines of sight (prism angle) from the left and right subject eyes EL and ER toward the visual target for the left eye 8L and the visual target for the right eye 8R. The accommodation reaction amount measurement step is executed in parallel with the control of the convergence distance L (±n Δ), measures the eye characteristics of the left and right subject eyes EL and ER with the objective measurement optical systems 6 and 7 when fusion is attempted with the left and right subject eyes EL and ER to the change of the convergence distance L (±n Δ), and acquires the objective refraction value as the objective measurement information. The determination step determines adaptation abilities of the left and right subject eyes EL and ER to stereoscopic vision based on the objective refraction value. For this reason, the ophthalmic examination method (FIG. 4) can evaluate whether the left and right subject eyes EL and ER have high adaptation abilities to stereoscopic vision in which a discrepancy between convergence and accommodation occurs. In addition, the ophthalmic examination method (FIG. 4) can perform training for improving the adaptation abilities to stereoscopic vision by utilizing the convergence distance control step and causing the left and right subject eyes EL and ER to repeatedly observe and habituate those having the convergence distances L (±n Δ) shifted.
(10) The ophthalmic examination method includes the subjective response acquisition step (step S6, step S8) of acquiring a subjective response related to fusion from the subject when fusion is attempted with the left and right subject eyes EL and ER with respect to a change in the convergence distance. The determination step determines adaptation abilities of the left and right subject eyes EL and ER to stereoscopic vision based on the subjective response and the objective refraction value. Therefore, when determining the adaptation abilities of the left and right subject eyes EL and ER to stereoscopic vision, the ophthalmic examination method (FIG. 4) can check whether fusion is subjectively achieved.

### Example 2

Example 2 is an example of prescribing a state in which the convergence distance L (±n Δ) changes in a stepless manner in a distance range from a position farther than the examination distance Lid to a position closer than the examination distance Lid. Note that, in Example 2, the "Entire Configuration of Apparatus", "Configuration of Optical System", and "Configuration of Controller" have the same configurations as those in FIGS. 1, 2, and 3 of Example 1, and thus, illustration and description are omitted.

[Configuration and Flow of Actions of Process of Evaluating Adaptation Abilities to Stereoscopic Vision (FIG. 10)] The flowchart of FIG. 10 describes the configuration and flow of actions of the process of evaluating the adaptation abilities to stereoscopic vision with the adaptation check portion 143. Note that steps S21, S22, S23, S24, S25, and S26 are the same processing steps corresponding to steps S1, S2, S3, S4, S5, and S6 in FIG. 4, respectively, and thus description thereof is omitted.

Step S27 is a prism angle change processing step in which the subjective examination processing portion 144 controls the rotation angle of the drive portion 122a to change the prism angle from the initial prism angle (0 Δ) or a prism angle of the previous time in a stepless manner with the examination distance (for example, 1 m) maintained. For example, the prism angle is changed from 0 Δ to +10 Δ in a stepless manner, and then is further changed from 0 Δ to -10 Δ in a stepless manner. For example, when the initial prism angle is 0 Δ, the prism angle is changed to -10 Δ in a stepless manner.

Step S28 is a subjective response step in which the subjective examination processing portion 144 acquires a subjective response related to fusion of the visual target for the left eye and the visual target for the right eye from the subject, when the fusion is attempted with the left and right subject eyes EL and ER with respect to the stepless change in the convergence distance. The subjective examination processing portion 144, as the subjective response, for example, when the convergence distance changes in a stepless manner, acquires a response as to whether visual targets are seen as one visual target by fusion at predetermined time intervals. The change of the prism angle may be temporarily stopped when the subjective response is prompted.

Step S29 is a final prism angle determination step in which the subjective examination processing portion 144 determines whether the prism angle reaches to the final prism angle (for example, ±10 Δ). While the subjective examination processing portion 144 determines NO in step S29, the process returns to step S27, and when the subjective examination processing portion determines YES in step S29, the process proceeds to step S33. Note that, in step S29, time required for the prism angle to reach the final prism angle at the stepless change speed may be set to determine whether the set time elapses.

Steps S27 and S29 correspond to the convergence distance control step of prescribing a state in which the convergence distance from the positions of the left and right subject eyes EL and ER to the convergence position where the two lines of sight intersect each other changes by control to change a prism angle from the left and right subject eyes EL and ER toward the visual target for the left eye and the visual target for the right eye. Steps S26 and S28 correspond to a subjective response acquisition step of acquiring a subjective response related to fusion from the subject when fusion is attempted with the left and right subject eyes EL and ER with respect to a change in the convergence distance.

Step S30 is a refractometry step in which the objective measurement portion 145 measures the eye characteristics of the left and right subject eyes EL and ER with respect to the change in the convergence distance with the objective measurement optical system and acquires the objective refraction value as the objective measurement information in time series. Step S31 is a refractometry value display step in which the objective measurement portion 145 displays objective refraction values (refractometry values) acquired in time series during the prism angle changes in a stepless manner. Step S32 is a time-series refractometry value comparison step in which the objective measurement portion 145 compares refractometry values in time series acquired while the prism angle changes in a stepless manner. These steps S30 to S32 correspond to the accommodation reaction amount measurement step of being executed in parallel with the control of the convergence distance, measuring the eye characteristics of the left and right subject eyes EL and ER with the objective measurement optical system when fusion is attempted with the left and right subject eyes EL and ER to the change of the convergence distance, and acquiring the objective refraction value as the objective measurement information.

Step S33 is a step in which the determination portion 146 determines adaptation abilities of the left and right subject eyes EL and ER to the stereoscopic vision based on the subjective response from the subjective examination processing portion 144 and the objective refraction value (time-series refractometry value) from the objective measurement portion 145. In step S33, the process proceeds to the end, when the determination portion 146 completes the determination of the adaptation abilities of the stereoscopic vision. This step S33 corresponds to a determination step of determining the adaptation abilities of the left and right subject eyes EL and ER to the stereoscopic vision based on the subjective response and the objective refraction value.

The flow of actions of each step is described as follows. In step S26, the process of FIG. 10 proceeds to step S27 to step S28 to step S29 after a subjective response related to fusion when the convergence distance and the examination distance are matched is acquired from the subject. In step S27, the subjective examination processing portion 144 controls the rotation angle of the drive portion 122a that consecutively changes the prism angle from the initial prism angle (for example, 0 Δ). In step S28, the subjective examination processing portion 144 acquires, from the subject, the subjective response related to the fusion when the prism angle is switched to the prism angle (for example, +1 Δ). In step S29, the subjective examination processing portion 144 determines whether the prism angle consecutively changes to the final prism angle (for example, +10 Δ) in the direction in which the convergence distance approaches, then changes to the initial prism angle (for example, 0 Δ) once, and then changes to the final prism angle (for example, -10 Δ) in the direction in which the convergence distance separates.

In the process of FIG. 10, in step S29, while the subjective examination processing portion 144 determines that no prism angle is changed to the final prism angle (for example, ±10 Δ), the flow from step S27 to step S28 to step S29 is repeated. In step S27, the subjective examination processing portion 144 controls the rotation angle of the drive portion 122a to switch the prism angle in a stepless manner. In step S28, while the prism angle is changed in a stepless manner, the subjective examination processing portion 144 acquires a subjective response related to fusion at a predetermined time interval from the subject.

In the process of FIG. 10, in step S25, when the subjective examination processing portion 144 matches the convergence distance with the examination distance, the objective measurement portion 145 starts the objective measurement in parallel with the subjective examination process. In the objective measurement, during the subjective examination process, the flow proceeding to step S30 to step S31 to step S32 is repeated. If the subjective examination process ends (YES in step S29), the objective measurement ends in accordance with the end of the subjective examination process. In step S30, the objective measurement portion 145 acquires the objective refraction values (refractometry values) in time series as the objective measurement information by the refractometry. In step S31, the objective measurement portion 145 displays the acquired time-series refractometry value. In step S32, the objective measurement portion 145 compares the time-series refractometry value.

In the process of FIG. 10, in step S29, when the subjective examination processing portion 144 determines that the prism angle reaches the final prism angle (for example, ±10 Δ), the process proceeds from step S29 to step S33. In step S33, the determination portion 146 determines adaptation abilities of the left and right subject eyes EL and ER to the stereoscopic vision based on the subjective response from the subjective examination processing portion 144 and the time-series objective refraction values from the objective measurement portion 145.

As described above, the flow of the processing action of evaluating the adaptation abilities to the stereoscopic vision is executed in parallel with the subjective examination process and the objective measurement and is a flow that merges with the objective measurement in step S33 when the subjective examination process ends. In step S33, for example, when a subjective response that the fusion by binocular vision is achieved is obtained irrelevant to the stepless change of the convergence distance and the time-series change of the objective refraction value is suppressed in a predetermined range, the determination portion 146 determines that the left and right subject eyes EL and ER have high adaptation abilities to stereoscopic vision. In step S33, for example, by changing the convergence distance in a stepless manner, when the subjective response that two visual targets are seen is obtained and no fusion by binocular vision is formed, or the time-series change of the objective refraction value exceeds the predetermined range, the determination portion 146 determines that the left and right subject eyes EL and ER have low adaptation abilities to stereoscopic vision. Furthermore, by obtaining time-series objective refraction values with respect to convergence stimulation, fluctuations in the objective refraction values can also be measured. For example, the determination portion 146 determines that the left and right subject eyes EL and ER have low adaptation abilities to stereoscopic vision when the frequency is analyzed, and the fluctuation range in a specific frequency band is large.

[Effect of Ophthalmic Examination Apparatus] (11) The subjective examination processing portion 144 prescribes a state in which the convergence distance L (±n Δ) changes in a stepless manner in a distance range from a position farther than the examination distance Lid to a position closer than the examination distance Lid by changing the angles between the lines of sight (prism angles) from the left and right subject eyes EL and ER toward the visual target for the left eye 8L and the visual target for the right eye 8R in a stepless manner. The objective measurement portion 145 measures the eye characteristics of the left and right subject eyes EL and ER in a state where the convergence distance L (±n Δ) changes in a stepless manner, and acquires objective refraction values obtained in time series as objective measurement information. Therefore, the subjective examination processing portion 144 can give convergence stimulation that changes in a stepless manner to the left and right subject eyes EL and ER. In addition, the objective measurement portion 145 can measure fluctuations in the objective refraction values indicating fluctuations in the accommodation reaction amounts of the left and right subject eyes EL and ER with respect to convergence stimulation that changes in a stepless manner.

The ophthalmic examination apparatus 100 and the ophthalmic examination method (FIGS. 4 and 10) of Examples 1 and 2 are described above with reference to the drawings. However, the specific configurations of the ophthalmic examination apparatus and the ophthalmic examination method of the present disclosure are not limited to Examples 1 and 2, and modifications, additions, and the like of the design are allowed without departing from the gist of the invention according to each claim of the claims.

The ophthalmic examination apparatus 100 of Examples 1 and 2 is an example of an objective measurement machine with a subjective examination function by an internal visual target integrally provided with a visual mark presentation function, a phoropter function, and an Auto Ref/Keratometry function. However, the ophthalmic examination apparatus is not limited to the apparatus configuration examples of Examples 1 and 2. The ophthalmic examination apparatus may be an example that is configured with, for example, a combined apparatus of a visual target projection device that presents a real visual target and prescribes a state in which the convergence distance changes and a device having a phoropter function and an auto refractometry function.

Examples 1 and 2 provide an example in which the ophthalmic examination apparatus 100 changes the angle between the lines of sight (prism angle) by changing a rotation angle by which the left measurement head portion 122L and the right measurement head portion 122R are rotated in opposite directions. The ophthalmic examination apparatus is not limited to the examples of the change in the angle between the lines of sight in Examples 1 and 2. For example, the ophthalmic examination apparatus may be an example of changing the angle between the lines of sight (prism angle) by inserting a prism lens at positions on the optical axis from the left and right subject eyes of the visual target projection system to the visual target presentation portion.

Examples 1 and 2 provide an example in which the determination portion 146 determines the adaptation abilities of the left and right subject eyes EL and ER to stereoscopic vision based on the subjective response acquired from the subject with the subjective examination processing portion 144 and the objective refraction value acquired with the objective measurement portion 145. However, the determination portion is not limited to the examples of the determination portion in Examples 1 and 2. For example, the determination portion may be an example of determining the adaptation abilities of the left and right subject eyes to stereoscopic vision based on only the objective refraction value.

Example 1 provides an example in which the subjective examination processing portion 144 performs control to change the angle between the lines of sight (prism angle) in a stepwise manner and acquires the average objective refraction value obtained by averaging the objective refraction values obtained a predetermined number of times in each step. Example 2 provides an example in which the subjective examination processing portion 144 changes the angle between the lines of sight (prism angle) in a stepless manner and acquires the objective refraction value obtained in time series. However, the subjective examination processing portion is not limited to Examples 1 and 2. The subjective examination processing portion may be an example of changing the angle between the lines of sight (prism angle) in a stepwise manner and acquiring the objective refraction value obtained in time series regardless of the step control.

The ophthalmic examination apparatus and the ophthalmic examination method of the present disclosure are also acceptable for the following points. (a) The range of the prism angle may be determined on the assumption of, for example, a 3D video to be viewed or an actual value on a head mounted display. Accordingly, adaptation abilities to a specific stereoscopic image can be determined. (b) The anterior segment image may be consecutively acquired during the measurement of the objective refraction value, so that alignment may be automatically performed when the alignment is deviated. (c) In the determination of the adaptation abilities of the left and right subject eyes EL and ER to the stereoscopic vision, low adaptation abilities to the stereoscopic vision may be determined when the anterior segment images may be acquired in time series or at the timing when the prism angle changes, and a near vision reaction in which the pupil contracts, for example, when the convergence distance becomes near occurs by a predetermined amount or more. (d) In the determination of the adaptation abilities of the left and right subject eyes EL and ER to the stereoscopic vision, an adaptation coefficient to the stereoscopic vision may be calculated by combining a plurality of determination criteria.

## Claims

1. An ophthalmic examination apparatus (100) comprising:
a visual target projection system (4) including a visual target presentation portion (41) that presents a fixed visual target to left and right subject eyes (EL, ER);
an objective measurement optical system (6, 7) that objectively measures eye characteristics of the left and right subject eyes; and
a controller that controls each portion of the apparatus, wherein
the fixed visual target is a visual target for a left eye (8L) and a visual target for a right eye (8R) that are to be respectively projected to the left and right subject eyes (EL, ER) and have a same pattern,
the visual target presentation portion (41) presents the visual target for the left eye (8L) and the visual target for the right eye (8R) to visual target positions that are separated from the left and right subject eyes (EL, ER) by a certain examination distance,
the visual target projection system (4) prescribes a state in which a convergence distance from positions of the left and right subject eyes to a convergence position where two lines of sight intersect each other is changed by changing angles of the lines of sight from the left and right subject eyes to the visual target for the left eye (8L) and the visual target for the right eye (8R), and
the controller (140) measures the eye characteristics of the left and right subject eyes (EL, ER) with the objective measurement optical system (6, 7) and acquires an objective refraction value as objective measurement information when fusion is attempted with the left and right subject eyes with respect to a change in the convergence distance.

2. The ophthalmic examination apparatus (100) according to claim 1, wherein
the ophthalmic examination apparatus is an objective measurement machine with a subjective examination function,
the controller (140) includes an adaptation check portion (143) that evaluates adaptation abilities of the left and right subject eyes (EL, ER), and the adaptation check portion (143) includes a subjective examination processing portion and an objective measurement portion (145),
the subjective examination processing portion (144) presents the visual target for the left eye (8L) and the visual target for the right eye (8R) to the visual target positions separated by the certain examination distance from the left and right subject eyes (EL, ER) and prescribes a state in which the convergence distance from the positions of the left and right subject eyes (EL, ER) to the convergence position where the two lines of sight intersect each other is changed by changing an angle between the lines of sight from the left and right subject eyes (EL, ER) toward the visual target for the left eye (8L) and the visual target for the right eye (8R), and
the objective measurement portion (145) executes a process in parallel with a process in the subjective examination processing portion (144), measures the eye characteristics of the left and right subject eyes, when fusion is attempted with the left and right subject eyes to the change of the convergence distance with the objective measurement optical system (6, 7), and acquires the objective refraction value as objective measurement information.

3. The ophthalmic examination apparatus (100) according to claim 2, wherein
the ophthalmic examination apparatus includes a left measurement head portion (122L), corresponding to the left subject eye (EL), equipped with the visual target for the left eye (8L) and a left measurement optical system (125L) and a right measurement head portion (122R), corresponding to the right subject eye (ER), equipped with the visual target for the right eye (8R) and a right measurement optical system (125R), and
the subjective examination processing portion (144) changes the angle between the lines of sight by controlling a rotation angle for rotating the left measurement head portion (122L) and the right measurement head portion (122R) in opposite directions.

4. The ophthalmic examination apparatus (100) according to claim 2, wherein
the subjective examination processing portion (144) acquires a subjective response from a subject when the fusion is attempted with the left and right subject eyes (EL, ER) with respect to the change in the convergence distance, and
the adaptation check portion (143) includes, in addition to the subjective examination processing portion (144) and the objective measurement portion (145), a determination portion (146) that determines adaptation abilities of the left and right subject eyes (EL, ER) to a stereoscopic vision based on the subjective response from the subject acquired with the subjective examination processing portion (144) and the objective refraction value acquired with the objective measurement portion (145).

5. The ophthalmic examination apparatus (100) according to claim 4, wherein
the determination portion (146) determines that the left and right subject eyes (EL, ER) have high adaptation ability to stereoscopic vision when a positive subjective response to fusion in binocular vision is obtained with respect to the change in the convergence distance, and a change width of the objective refraction value is suppressed to a predetermined range.

6. The ophthalmic examination apparatus (100) according to claim 4, wherein
the determination portion (146) determines that the left and right subject eyes (EL, ER) have low adaptation ability to stereoscopic vision when a negative subjective response to fusion in binocular vision is obtained with respect to the change in the convergence distance, and a change width of the objective refraction value exceeds a predetermined range.

7. The ophthalmic examination apparatus (100) according to claim 2, wherein
the adaptation check portion (143) includes, in addition to the subjective examination processing portion (144) and the objective measurement portion (145), a determination portion (146) that determines the adaptation abilities of the left and right subject eyes (EL, ER) to stereoscopic vision based on the objective refraction value acquired with the objective measurement portion (145), and
the determination portion (146) measures an amplitude that is a fluctuation range of a refractive value characteristic of the objective refraction value acquired with the objective measurement portion (145) with respect to a time axis and determines that the left and right subject eyes (EL, ER) belong to a fatigue group, when a fluctuation frequency of the refractive value characteristic is a low frequency band, and the amplitude is a determination threshold or more.

8. The ophthalmic examination apparatus (100) according to any one of claims 2-7, wherein
the subjective examination processing portion (144) prescribes a state in which the convergence distance changes in a stepwise manner in a distance range from a position farther than the examination distance to a position closer than the examination distance by changing the angle between the lines of sight from the left and right subject eyes (EL, ER) toward the visual target for the left eye (8L) and the visual target for the right eye (8R) in a stepwise manner, and
the objective measurement portion (145) measures the eye characteristics of the left and right subject eyes (EL, ER) a predetermined number of times in each step of the convergence distance and acquires an average objective refraction value obtained by averaging objective refraction values obtained a predetermined number of times in each step as objective measurement information.

9. The ophthalmic examination apparatus (100) according to any one of claims 2-7, wherein
the subjective examination processing portion (144) prescribes a state in which the convergence distance changes in a stepless manner in a distance range from a position farther than the examination distance to a position closer than the examination distance by changing the angle between the lines of sight from the left and right subject eyes (EL, ER) toward the visual target for the left eye (8L) and the visual target for the right eye (8R) in a stepless manner, and
the objective measurement portion (145) measures the eye characteristics of the left and right subject eyes (EL, ER) in a state in which the convergence distance changes in a stepless manner and acquires an objective refraction value obtained in time series as objective measurement information.

10. An ophthalmic examination method in which
a visual target projection system (4) that includes a visual target presentation portion (41) that presents a fixed visual target to left and right subject eyes (EL, ER),
an objective measurement optical system (6, 7) that objectively measures eye characteristics of the left and right subject eyes (EL, ER), and
a controller (140) that controls each portion of apparatus are included, the method causes the controller (140) to perform:
a complete correction prescription step of assuming the left and right subject eyes as a state in which a complete correction value in subjective examination is prescribed;
a visual target presentation step of presenting a visual target for the left eye (8L) and a visual target for the right eye (8R) having a same pattern in the visual target presentation portion (41) at visual target positions separated from the left and right subject eyes (EL, ER) by a certain examination distance following the prescription of the complete correction value;
a convergence distance control step of controlling an angle between lines of sight from the left and right subject eyes (EL, ER) toward the visual target for the left eye (8L) and the visual target for the right eye (8R) to prescribe a state in which a convergence distance from positions of the left and right subject eyes (EL, ER) to a convergence position where the two lines of sight intersect each other is changed;
an accommodation reaction amount measurement step of being executed in parallel with the control of the convergence distance and measuring the eye characteristics of the left and right subject eyes (EL, ER) with the objective measurement optical system (6, 7) when fusion is attempted with the left and right subject eyes (EL, ER) to the change of the convergence distance to acquire an objective refraction value as objective measurement information; and
a determination step of determining adaptation abilities of the left and right subject eyes (EL, ER) to stereoscopic vision based on the objective refraction value.

11. The ophthalmic examination method according to claim 10, further comprising
a subjective response acquisition step of acquiring a subjective response related to the fusion from a subject when the fusion is attempted to a change in the convergence distance with the left and right subject eyes (EL, ER), wherein
the determination step determines adaptation abilities of the left and right subject eyes (EL, ER) to stereoscopic vision based on the subjective response and the objective refraction value.
